# EUROPEAN PATENT APPLICATION

(11) **EP 4 799 554 A1**
(43) Date of publication of application: **02.09.2026**
(21) Application number: 25160735.4
(22) Date of filing: 27.02.2025
(51) Int. Cl.: A61B 3/16, A61B 5/03, A61B 5/00

(54) **NON-INVASIVE INTRAORBITAL AND INTRACRANIAL PULSE WAVE MONITOR**

(71) Applicant: ScienceForBrain UAB, 44145 Kaunas (LT)
(72) Inventor: RAGAUSKAS, Arminas, 51362 Kaunas (LT); PETKUS, Vytautas, 48300 Kaunas (LT); CHALECKAS, Edvinas, 50374 Kaunas (LT); BARTUSIS, Laimonas, 53363 Kaunas district (LT); PUTNYNAITÉ , Vilma, 50172 Kaunas (LT); HAMARAT, Yasin, 53363 Kaunas district (LT); DEIMANTAVICIUS, Mantas, 49212 Kaunas (LT); LAGAUSKAS, Martynas, 66142 Druskininkai (LT); KARALIUNAS, Andrius, 48405 Kaunas (LT)
(74) Representative: Sayettat, Julien Christian

(57) **Abstract**

The invention is a non-invasive intracranial pulse wave monitor and method for measuring pulsating cerebrospinal fluid pressure in the subarachnoid space of the optic nerve in real-time.

## Description

### FIELD OF THE INVENTION

The field of the invention is a device that permits the non-invasive measurement and monitoring of intraorbital and intracranial pressure in real time and real time monitoring of intracranial compliance in humans.

### BACKGROUND OF THE INVENTION

In cases of severe traumatic brain injuries, non-invasive real-time monitoring of intracranial compliance is currently not possible in clinical practice. The inventive device and method solve the problem of non-invasive intracranial compliance monitoring by using an intracranial pressure monitor to measure in real-time and real-time algorithms for morphological analysis and complexity analysis of recorded pulse waves.

Intracranial pressure (ICP) pulse waveform and its parameters provide valuable diagnostic information about brain conditions. The importance of intracranial pressure pulse wave morphology, which provides additional information beyond the absolute ICP value, was recognized decades ago. Intracranial compliance can be reduced after traumatic brain injury, hemorrhagic stroke, and other pathological brain conditions, even when ICP is within the normal range. The ratio between the second and first peaks (P2/P1) extracted from the ICP pulse wave is related to intracranial compliance. Although the diagnostic information extracted from the ICP pulse waveform is valuable, currently it has only been used in intensive care units for patients with implanted invasive ICP sensors. In situations such as diagnosing normal-tension glaucoma, performing cardiac surgery, organ transplantation, or in aero-space medicine, where invasive ICP sensors cannot be implanted into patients' brains, there is a need for non-invasive monitoring of ICP pulse waves and changes.

Several studies have shown that monitoring skull pulsations, brain photoplethysmographic signals, the dielectric properties of the head, and ultrasonic time-of-flight changes in brain tissue have the potential to estimate characteristics related to intracranial compliance non-invasively. The routine clinical applications of these recent technologies are still unclear. The MRI-based studies analysed in a review paper indicate that the amplitude of brain tissue pulsations ranges from 0.04 mm to 0.8 mm. Recent MRI 3D models can visualize cardiac-induced CSF and brain tissue pulsations in three dimensions over time. However, MRI technology has limitations when physiological multimodal brain monitoring is used in intensive care units.

Traditional invasive methods for ICP pulse wave monitoring pose significant risks, highlighting the need for non-invasive alternatives. Invasive intraventricular catheter systems continue to be used as the standard for ICP pulse wave monitoring. However, because they are invasive their high risk/benefit ratio restricts their use to patients with traumatic brain injuries or certain neurological conditions. Consequently, there is an increasing demand for a non-invasive technique to monitor ICP pulse waves and intracraniospinal compliance. However, experimental studies have shown that the amplitude of the ICP pulse wave depends primarily on the mean value and the pulse amplitude of the cerebral blood volume, depending on the pathology.

There is also a need for a simple noninvasive monitoring device that can be easily assembled and create hermetically sealed chambers for accurate pressure measurement through a patient's eyes and can be quickly customized to fit a patient's unique facial structure.

Intracranial pressure (ICP) pulse wave morphology, including the ratios of three peaks (P1, P2, P3), offers valuable insights into brain conditions. The ICP pulse waveform is characterized not only by its amplitude but also by the presence of three distinct peaks: P1, P2, and P3. Other parameters include peak appearance time, rise time coefficient, downward coefficient, wave duration, area under curve, etc. Overall, 24 metrics can be extracted using an algorithm termed Morphological Clustering and Analysis of ICP Pulse (MOCAIP).

### BRIEF SUMMARY OF THE INVENTION

The invention is an innovative non-invasive intracranial/intraorbital pulse wave monitor and method for non-invasively measuring ICP. The intracranial pressure pulse waves sensor of this monitor is completely passive, i.e. does not radiate any physical signals to the eye, orbit or intracranial medium, and does not add any pressure to the eye or orbit. It consists of goggles with two cups, each cup attached to a closed eyelid of a patient, and is hermetically sealed and connected to the facial tissue around the eye. The inner volume of each cup of the goggles is filled with a non-compressible liquid (degassed water or physiological liquid), in which a digital pressure sensor is placed. The liquid is isolated from the closed eyelids with a non-allergenic elastic plastic film 50 microns thick. The pressure sensor is connected to a medical microelectronic Bluetooth transmitter to transmit the pressure signals. The pressure sensor transmits real-time signals of monitored pulse waves to a laptop. The pressure sensor signal is displayed on the screen of the laptop, filtered, and undergoes pulse wave selection procedures against the background of slow and respiratory waves. Subsequently, algorithms are employed to recognize and classify these waves. The intracranial pressure wave measurement allow for real time assessment of a patient's ICP and autoregulation states.

### BRIEF DESCRIPTON OF THE FIGURES

Figure 1 is an illustration of a non-invasive pulse wave monitor placed on a patient.
Figure 2A(1)-2B(2) is a set of graphs showing measurements of a patient's non-invasively recorded pulse waves and invasively recorded intracranial pressure.
Figure 3A(1)-3B(2) is a set of graphs showing measurements of patients non-invasively recorded pulse waves.
Figure 4 is an illustration of a non-invasive pulse wave monitor placed on a patient.
Figure 5 is a graph of an intraorbital/intracranial pressure pulse wave.
Figure 6 is an illustration of a non-invasive two channel pulse wave monitor placed on a patient.
Figure 7 is a set of graphs showing measurements of a patient's non-invasively recorded intracranial pressure waves.
Figure 8A(1)-8B(2) is a set of figures showing the non-invasive pulse wave monitor and ultrasound triplex scanner placed on a patient and a set of figures showing ultrasound scans.
Figure 9A-9B is a set of graphs showing the intracranial pressure pulse waves of a patient.
Figure 10 is a non-invasive pulse wave monitor placed on a patient.
Figure 11A-11B is a set of graphs showing measurements of a patient's non-invasively recorded intracranial pressure waves.
Figure 12a is a top view of one embodiment of the intracranial pressure monitor.
Figure 12b is a back view of one embodiment of the intracranial pressure monitor.
Figure 13 is a top exploded view of one embodiment of the intracranial pressure monitor with the disposable goggle system separated from the right and left side sensors.
Figure 14 is a top view of one embodiment of the disposable goggle system.
Figure 15 is a top exploded view of one embodiment of the disposable goggle system.
Figure 16 is a front view of one embodiment of the right side sensor assembly.
Figure 17 is a top perspective exploded view of one embodiment of the right side sensor assembly.
Figure 18 is a front view of one embodiment of the left side sensor assembly.
Figure 19 is a top perspective exploded view of one embodiment of the left side sensor assembly.
Figure 20A-20B are graphs comparing non-invasive and invasive measurement of ICP pulse waves.
Figure 21 are graphs comparing non-invasive and invasive measurement of ICP pulse waves.
Figure 22 is a graph comparing non-invasive and invasive measurement of ICP pulse waves.
Figure 23 is a graph comparing non-invasive and invasive measurement of ICP pulse waves.
Figure 24 are graphs comparing non-invasive and invasive measurement of ICP pulse waves.
Figure 25 is a graph comparing non-invasive and invasive measurement of ICP pulse waves
Figure 26A-26B are graphs comparing non-invasive and invasive measurement of ICP pulse waves
Figure 27A-27C are graphs showing pressure pulse waive recordings during a Valslava maneuver.
Figure 28A-28C are graphs showing pressure pulse waive recordings during a Queckenstedt test.
Figure 29 is a graph showing pressure pulse waves during a Transient Hypoemic/Hyperemic Response test.
Figure 30 are graphs comparing non-invasive and invasive measurement of ICP pulse waves.
Figure 31A-31B are graphs of subjects measured ICP pulse waves.
Figure 32A-32D are graphs of averaged measured ICP pulse waves.
Figure 33A-33B are ultrasound images both before and after jugular vein compression
Figure 34A-34B are ultrasound images during an ultrasound guided carotid artery compression test.
Figure 35 is a front perspective view of a intracranial pressure monitor.
Figure 36 is a rear view of an intracranial pressure monitor with out the headband or silicon film.
Figure 37 is a rear view of the left and right sensor assemblies.
Figure 38 is a side perspective view of the locking ring of the left sensor assembly.
Figure 39 is a rear view of the goggle assembly without the head strap.
Figure 40 is a front view of the goggle assembly without the head strap.
Figure 41 is a top view of the right and left sealing covers, flexible connector and O-rings partially assembled.
Figure 42 is a front view of a set of flexible connectors of different sizes.

### DETAILED DESCRIPTION OF THE INVENTION

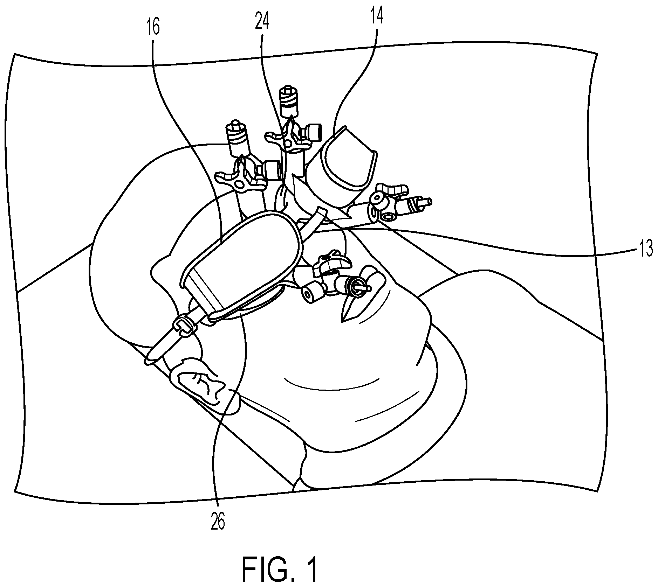

The invention includes a system for the intracranial pressure wave monitoring through a patient's closed eyelid. FIG. 1 shows one embodiment of the invention, a non-invasive, wireless, and fully passive sensors that can be placed over both closed eyelids and gently secured with a band around the back of the head of a patient. An additionally embodiment not show is a monitor with a single sensor used in situations where only one eye can or needs to be used for ICP dynamic monitoring.

**FIG. 1** shows two cups **24, 26** that touch the closed left and right eyelids are designed to be disposable, and sensor modules can be easily connected and disconnected on top of them. Bottom part of the cups are made of thin (50 µm) non-allergic elastic film which directly touches the closed eyelid. When the sensor modules **14, 16** are hermetically fixed on top of the cups, the inner volume of the cups is filled with a non-compressible liquid, preferably water. The digital pressure sensors on both the left and right sides are installed so that their pressure ports, preferably stainless steel, protrude into the cup, making direct contact with the liquid. The right sensor module **16** contains a digital pressure sensor, which is connected to the left module's **14** main control board via a signal cable **13.** The digital pressure sensor, integrated with hardware and software monitors ICP waves and ICP changes by detecting three peaks in the pressure signal of the non-compressible liquid, which result from slight eyeball movements caused by pulsatile and dynamic variations in ICP(t). The invention also contemplates embodiments that once powered on, can record and wirelessly transmit real-time pressure signals to a data acquisition application designed for both laptops and smartphones.

The invention is an innovative intracranial/intraorbital pulse wave monitor and method. The intracranial pressure pulse waves sensor of this monitor is completely passive, i.e. does not radiate any physical signals to the eye, orbit or intracranial medium, and does not add any pressure to the patient's eye or orbit. It consists of goggles attached to the closed eyelids, which are hermetically sealed and connected to the facial tissue around the eye. The inner volumes of the goggles are filled with a non-compressible liquid (degassed water or physiological liquid), in which a digital pressure sensor is placed. The liquid is isolated from the closed eyelids by a non-allergenic elastic plastic film of 50 microns. Preferably, the elastic plastic film is a silicon membrane and should be approximately 40-50 microns thick. Because of the thinness of the film the goggle system can only be used one time without the performance degrading. The goggle system is designed as a single use component. The pressure sensor is connected to a medical microelectronic Bluetooth transmitter to transmit the pressure signals. The pressure sensor transmits real-time signals of monitored pulse waves to a laptop or mobil device like a smart phone or pad. The pressure sensor signal is displayed on the screen of a laptop, filtered, and undergoes pulse wave selection procedures against the background of slow and respiratory waves. Subsequently, algorithms are employed to recognize and classify these waves. A kNN algorithm trained on healthy volunteers and normal tension glaucoma patient's waveform data was used to detect pathological pulse waves associated with normal tension glaucoma disease. Other commercially available classification algorithms could also be used to process the signals from the sensor's to identify and filter the wave forms.

The non-invasive monitor is absolutely safe for the patient because it is passive and does not add pressure to the eye and orbit. The operational principle relies on the hydrodynamic transmission to the pressure sensor of the monitor of pulsating cerebral spinal fluid (CSF) pressure in the subarachnoid space of the optic nerve. This transmission occurs through a non-compressible liquid and the substantially non-compressible tissues of the eyeball. Measurement of pulsating CSF pressure transmission through the eyes is technologically possible using the inventive pressure monitor that allows for the measuring, and recording the shape of intracranial pressure pulse waves with morphological features and characteristic complexity in a non-invasive manner, using a passive and patient-safe sensor. The invention permits recording the shape of intracranial pulse waves in both brain hemispheres passively, non-invasively and hemispheres independently. This creates a previously impossible opportunity to observe dynamic processes in an injured hemisphere compared to processes in the healthy hemisphere of a patient. The invention allows for a real time comparison of an injured hemisphere with a healthy hemisphere of a patient with traumatic brain injuries, regrowing tumors, or other abnormalities.

The principle of operation of the invention is based on the fact that CSF pulsation in the subarachnoid space of the optic nerve causes an associated spatial pulsation of the eyeball. The amplitude of such pulsation, measured using dynamic OCT technology which has limited resolution and is about 7.8 ± 1.3 micrometers. The resolution of OCT technology is limited - the minimum size of one pixel in the image is 1.95 micrometers. This resolution does not allow recording the morphological features and complexity of the intracranial/intraorbital pulse wave. The invention allows recording of the intracranial/intraorbital pulse waveforms with much better resolution than OCT technology and close or even better resolution compared to state-of-the-art invasive intracranial pressure monitors. The invention provides 100 measured pressure data points in 1 second duration single pulse wave recoded forms from the left and right eyes.

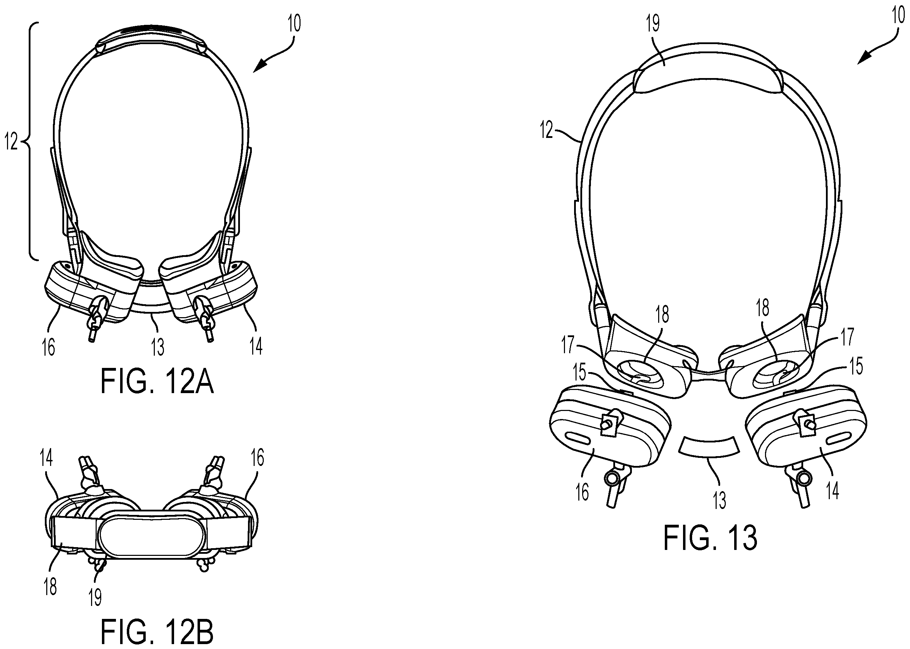

**FIG. 12A** is a top view of one embodiment of the novel intracranial pressure monitor **10** comprising a disposable goggle system **12,** a left side sensor assembly **14** and a right side sensor assembly **16** attached to the disposable goggle system **12** for positioning over the eyes of a patient. **FIG. 12B** shows a rear view of the intracranial pressure monitor **10** including with the disposable goggle system **12** including an adjustable strap **18** and head back support **19** for fitting around a patient's head. **FIG. 13** shows the intracranial monitor **10** with the left side sensor assembly **14** and the right side sensor assembly **16** unlocked and separated from the disposable goggle system **12.** Tabs **15** on that extend from the outside of the left side and right side sensor assemblies **14, 16** can be inserted in the ramps **17** on the inside of the right and left sealing covers **20, 22** of the goggle system **12** to hermetically seal the sensor assemblies **14, 16** to the goggle system 12.

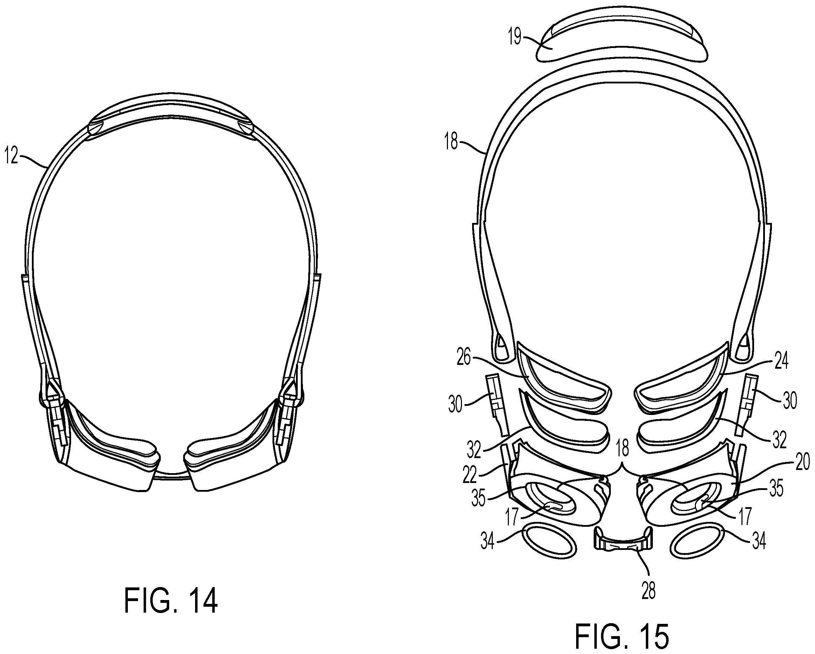

**FIG. 14** shows the top view of an assembled disposable goggle system **12.** **FIG. 15** shows an exploded view of the components of the disposable goggle system **12.** The disposable goggle system **12** is comprised of a right front sealing cover **20,** a left front sealing cover **22,** a back right sealing cover **24,** a back left sealing cover **26,** a flexible connector **28** for connecting the right front and left front sealing covers, **20, 22,** band connectors **30** for connecting the adjustable strap **18** to the right front and left front sealing covers **20, 22.** A silicon membrane **32** is positioned between each of the right front and left front sealing covers **20, 22** and the respective back sealing covers **24, 26** with a hermetic water tight seal around the exterior of the silicon membrane **32** and the right and left front sealing covers **20, 22** and the respective back sealing covers **24, 26.** When assemble and the front sealing cover, back sealing cover and silicon membrane partially form a sealed chamber into which fluid such as water can be injected. Water is injected into the chamber through each of the pressure assemblies, using a standard medical syringe to precisely control the fluid volume. The fluid pressure in the chamber is maintained as low as possible. Typically, the optimum pressure in the chamber is between 1.0 mmHg and 3.0 mmHg. The mechanical contact between the closed eyelid and the elastic film sometimes cannot be achieved if the pressure in the water tight chamber is too low. In such situations a weak pulse wave signal will be observed during monitoring. In order to get a good signal it is preferable to increase the pressure in the chamber 1 or 2 mmHg. This improves the mechanical contact with the closed eyelid. During initial setup of pressure adjustment the operator controls both the pressure in the goggles and pulse wave signals.

The disposable goggle system also includes O-rings **34,** which fit in grooves **35** on the front side of the right and left front sealing covers **20, 22.** The O-rings **34** from a water tight seal between the disposable goggle system **12** and the left side sensor assembly **14** and right side sensor assembly **16** when they are attached to the disposable goggle system **12.** In one embodiment the disposable goggle system **12** is attached to the left and right side sensor assemblies **14, 16** by twisting inclined tabs **15** extending on the sensor assemblies **14, 16** into inclined ramps **17** positioned in the side of openings **18** of the front sealing covers **20, 22** of the goggle system **12.** The twisting action causes the components to be pulled together forming water tight seals. Other attaching or sealing arrangements could also be used.

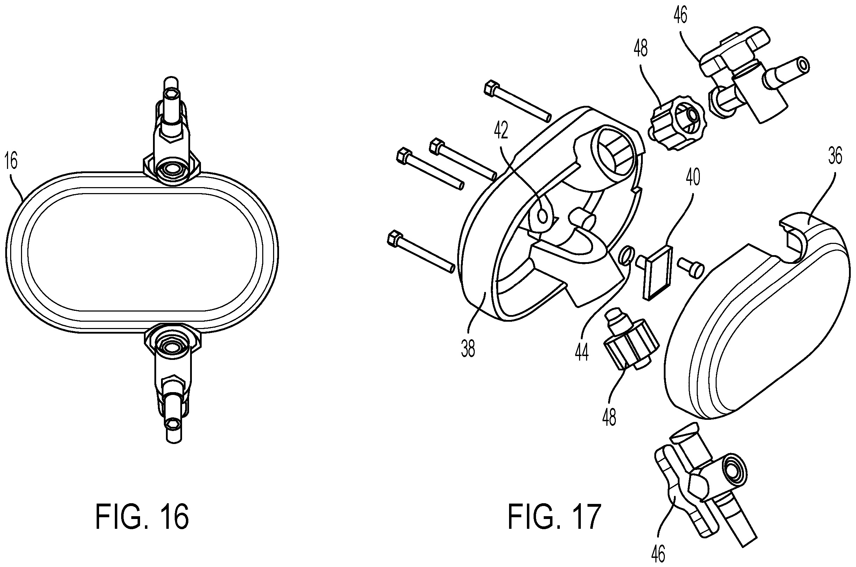

**Figure 16** is a front view of one embodiment of the right side sensor assembly **16.** **Figure 17** is a top perspective exploded view of one embodiment of the right side sensor assembly **16.** The right side sensor assembly is comprised of a front right cover **36,** a back right cover **38,** a pressure sensor **40** positioned between the front right cover **36** and back right cover **38** and extending through an outlet opening **42** in the back right cover **38.** There is a seal **44,** such as an o-ring, for forming a water tight seal and sealing the area around the pressure sensor **40** that extends through the back right cover **38.** The right side sensor assembly **16,** also includes two valves **46** one at the top of the assembly and one at the bottom of the assembly for allowing for the control of water, or another substantially noncompressible liquid, into and out of the intracranial pressure monitor **10.** The valves **46** can include Luer locks and can be attached to male luer integral locking rings **48** integral to the back right cover **38** for hermetically sealing the connection between the valve **46** and the back right cover. In use the valve **46** is connected to a standard medical grade plastic tube for filling the chambers with water. A syringe is used to inject water into the rights sensor assembly through the tube and into the valve **14.** The injected water continues through the right side assembly and exits through an outlet opening **42** in the back cover into the hermetically sealed chamber in the goggle system **12.** The water or fluid is injected through the left side sensor assembly in a similar manner. The tube which is connected to a water source such as a syringe or other liquid source allows for the flow of the liquid into the chamber(s) within the intracranial pressure monitor.

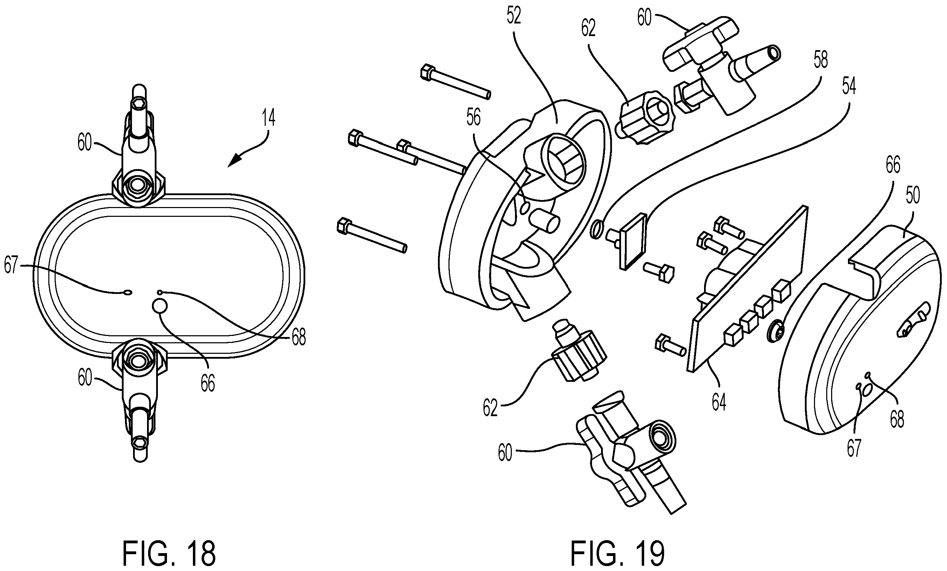

**Figure 18** is a front view of one embodiment of the left side sensor assembly. **Figure 19** is a top perspective exploded view of one embodiment of the left side sensor assembly **14.** The left side sensor assembly is comprised of a front left cover **50,** a back left cover **52,** a pressure sensor **54** positioned between the front left cover **50** and back left cover **52** and extending through an opening **56** in the back left cover **52.** There is a seal **58,** such as an o-ring, for forming a water tight seal and sealing the area around the pressure sensor that extends through the back left cover **52.** The left side pressure sensor assembly **14,** also includes two valves **60** one at the top of the assembly and one at the bottom of the assembly for allowing for the control of water, or another substantially noncompressible liquid, into and out of the intracranial pressure monitor **10.** The valves **60** can include Luer locks and can be attached to male luer integral locking rings **62** integral to the back left cover **52** for hermetically sealing the connection between the valve **46** and the back left cover **52.** In use the valve **46** is connected to a tube which is connected to a water source or other liquid source for that allows for the flow of the liquid into the chamber(s) within the intracranial pressure monitor.

The left sensor assembly **14** also includes a PCB board **64** for controlling the intracranial pressure monitor **10.** The PCB board **64** registers and transmits pressure readings from the pressure sensors using a Bluetooth low-energy network processor that transmits raw pressure data from both left and right pressure sensors by pairs of digital pressure measurement results to an external data acquisiton application (laptope. The sensor assembly also includes a battery for power supply to power the PCB board **64.** While a 3V CR2032 battery has been used other types of battery power sources could also be used. The PCB boardcon attached to the PCB board **64** and extending out through a hole in the front left cover **50** is a push button switch 66. The push button switch is operated manually by the finger of an operator to turn on and activate the device. Once the button **66** is pushed and immediately release the PCB board **64** is powered on and the pressure monitor 10 itself can operate. To turn off the pressure monitor **10** the operator pushes the button **66** and holds is for 2 seconds then releases the button **66.** When the button **66** is pressed and immediately released, powers on, is indicated by a blinking blue LED visible through the small hole **67.** When the monitor **10** is paired via Bluetooth with another device (such as a laptop or smartphone), the blue LED stops blinking and remains constantly lit. A second hole **68** hole, just above the power button, also serves as an indicator. When a red LED is visible through this hole **68,** it indicates that the battery is low and needs replacement.

The right side and left side sensor assemblies include high precision sensors that allow for precise measurements and superior real time information gathering.

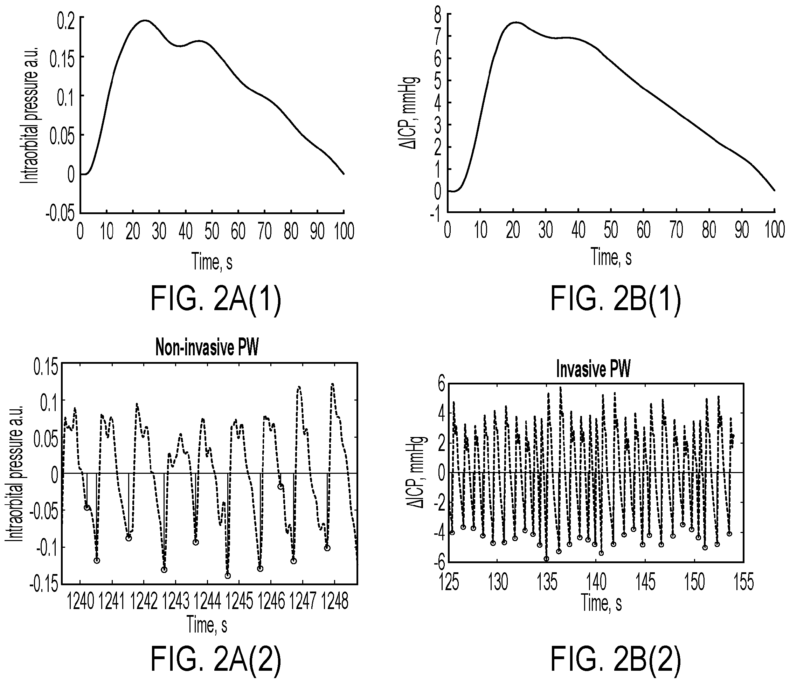

Comparative clinical studies using the invention on intensive care patients, featuring invasive intracranial pressure sensors implanted in the ventricle or parenchyma of the brain, have been carried out. One study involved the simultaneous monitoring of non-invasive and invasive pulse waves of intracranial pressure, seven patients were included after neurosurgery for subarachnoid hemorrhage, following neurosurgery for a ruptured intracranial aneurysm, and after the removal of a meningioma. Other studies of healthy volunteers (12 subjects) were also carried out. The results of the conducted tests are shown in **Figs 2** through **11****.** It is important to note that none of the active methods known to those of skill in the art (ultrasound, radio waves, electrical signals, near-infrared spectroscopy, etc.) achieve such a high correlation between non-invasively and invasively recorded pulsating CSF fluid waveforms as demonstrated using the inventive method and monitor. The non-invasively recorded wave forms with the highest amplitude have the best correlation. Amplitude of ICP pulse waves is modulated by the respiratory waves. This means that in each cycle of respiration at least one pulse wave with the highest amplitude correlates with the invasively recorded ICP(t) pulse wave at the same time.

**Fig. 2****.** shows the measurments for a patient after surgery for subarachnoid hemorrhage (SAH). The mean intracranial pressure (ICP) was 17.55 mmHg, the mean amplitude of ICP pulse waves was 10.20 mmHg. Shown in **Fig 2A1** is a non-invasively recorded pulse wave after initial signal processing shown in **Fig. 2A2** eliminating slow and respiratory waves; **Fig 2** **B1** shows an invasive ICP pulse wave after processing the original signal shown in **Fig 2** **B2** by eliminating slow and respiratory waves. Invase ICP wave was recorded with the Raumedic Neurovent-PTO ICP sensor implanted in the brain parenchyma. Correlation coefficient between the measured invasive and non-invasive pulse wave is R=0.993.

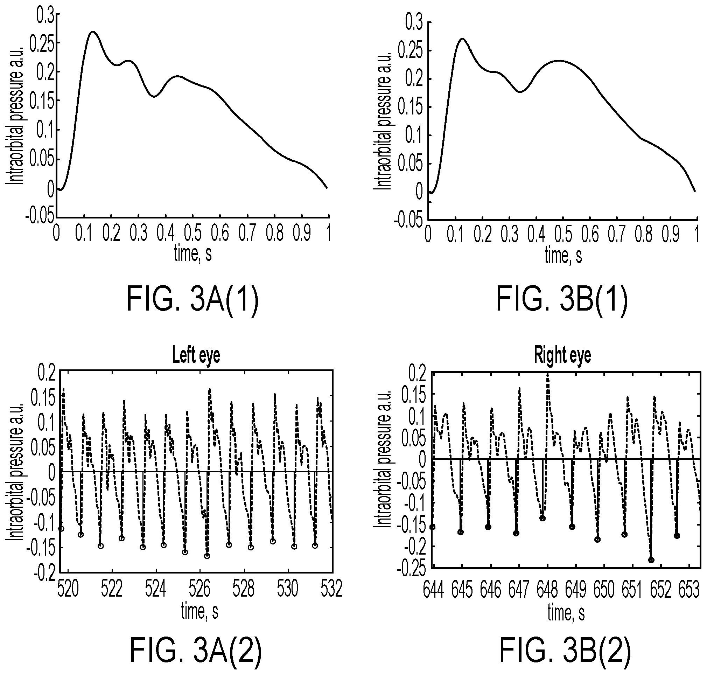

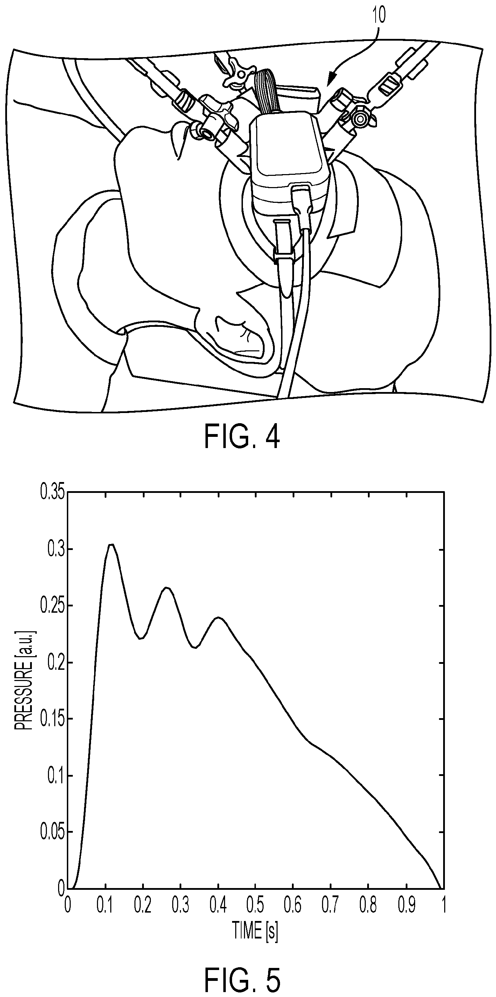

**Fig. 3****.** Shows pulse waves recorded during the test of a healthy volunteer laying in a supine body position using the inventive non-invasive intracranial pulse wave monitor. The curves in **Figs 3A2** and **3B2** represent non-invasively recorded pulse waves after elimination of respiratory and slow waves. The correlation coefficient R between the pulse waves of the left (**Fig 3A1**) and right (**Fig. 3B1**) eyes is equal to R=0.974. **Fig. 4****.** Illustrates a healthy volunteer in a supine body position with inventive monitor placed on the volutunteers closed eyelids. **Fig. 5****.** Shows the intraorbital/intracranial pressure pulse wave acquired from the left hemisphere of a healthy volunteer placed in a supine position using the non-invasive intracranial pressure monitor illustrated in **Fig. 4****.** Raw signals were recorded then, they were processed to remove intracranial slow and respiratory waves.

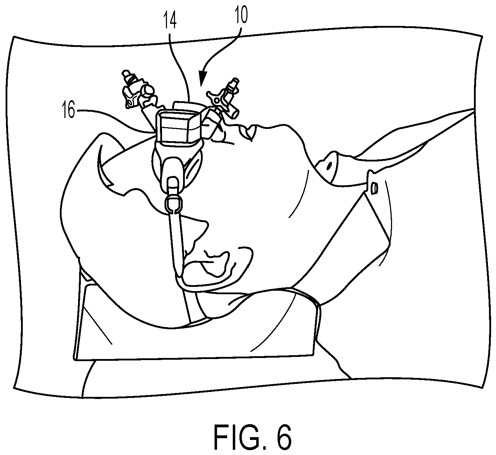

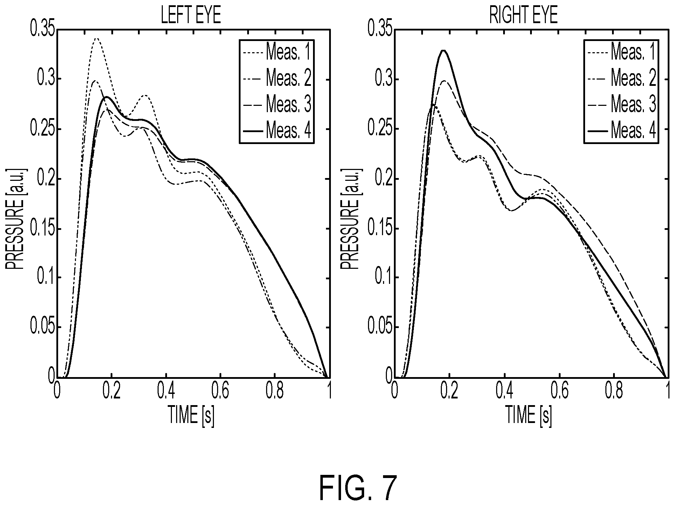

**Fig. 6****.** Illustrates a healthy volunteer in a supine body position with non-invasive two channels inventive monitor placed on the closed eye lids. **Fig. 7****.** Shows the intracranial pressure pulse waves acquired from the left and right hemispheres of a healthy volunteer using the non-invasive one embodiment of the pressure monitor illustrated in **Fig. 6****.** Measurements were performed on different days for the same healthy volunteer to check the repeatability of the signals. The calculated correlation coefficients between Meas. 1 and Meas. 2, Meas. 1 and Meas. 3, and Meas. 1 and Meas. 4 were R=0.997, R=0.917, and R=0.924 for the left eye, respectively. The calculated correlation coefficients between Meas. 1 and Meas. 2, Meas. 1 and Meas. 3, and Meas. 1 and Meas. 4 were R=0.999, R=0.948, and R=0.953 for the right eye, respectively. The correlation using the invention is substantially higher than the correlation in the range of 0.7-0.8 that is typically the best that can be achieved using the existing measurement methods.

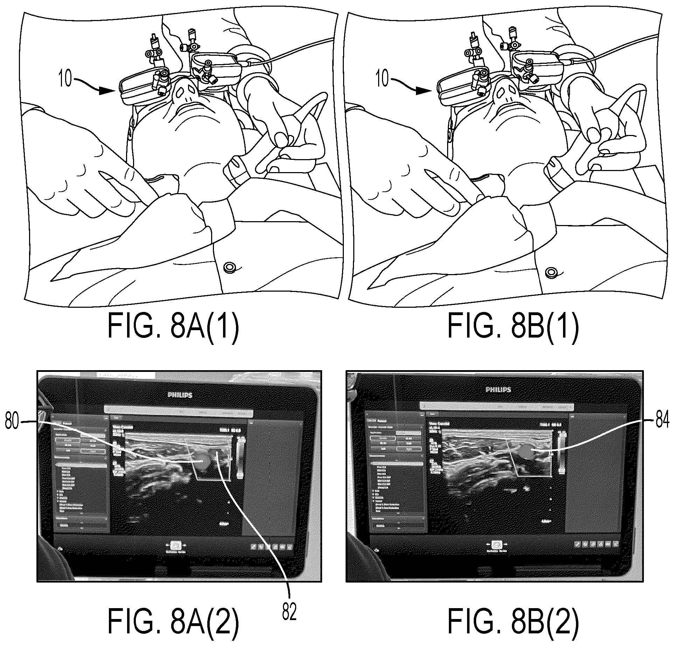

**Fig. 8****.** Shows a patient in a supine body position with the inventive monitor placed on the closed eyelids, during the ultrasound-guided jugular veins compression test (Queckenstedt's maneuver). **FIG 8A1** shows a patient with an ultrasound triplex scanner placed the patient's neck before compression of jugular veins and **FIG 8A2** is a the display image of the output of the ultrasound sensors, with coded external carotid artery blood volume **80** and coded jugular vein's blood volume **84** are visible on the screen of a PHILIPS EPIQ Elite ultrasonic scanner. In **FIG 8B1** the patient is in the supine body position with the inventive monitor placed on the closed eyelids during the compression of jugular veins. **FIG 8B2** is a display image of the output of the ultrasound sensors during compression of the jugular veins with only external corotid artelry blood volume **84** being visible while no blue coded jugular vein's blood volume is visible on the screen.

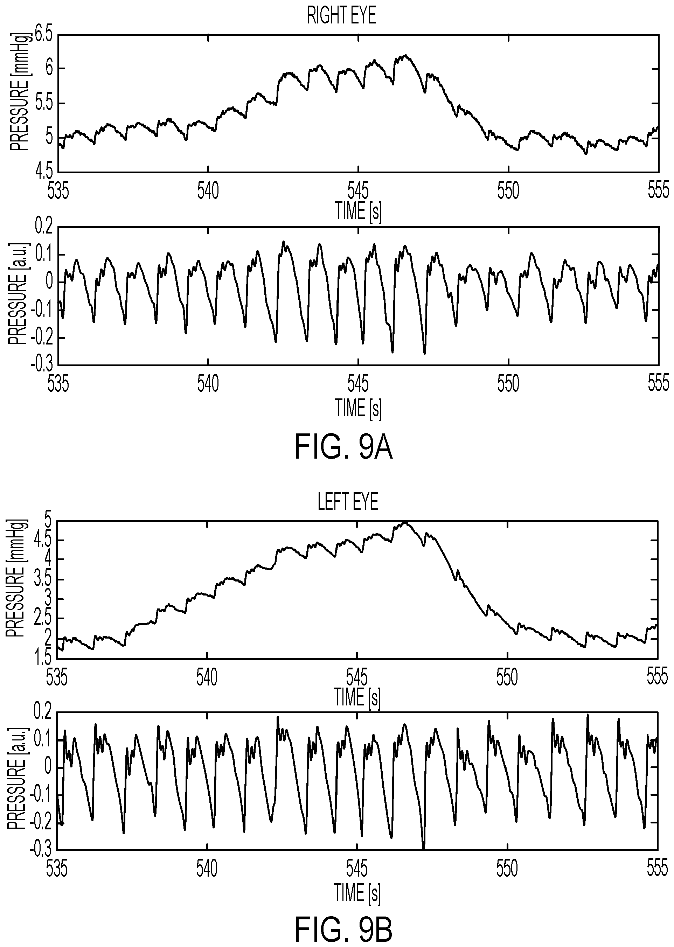

**Fig. 9****.** Shows the intracranial pressure pulse waves acquired from the left eye, right brain hemisphere (A) and the right eye, left brain hemisphere (B) of a healthy volunteer during a Queckenstedt maneuver using the inventive non-invasive pressure monitor illustrated in **Fig. 8****.**

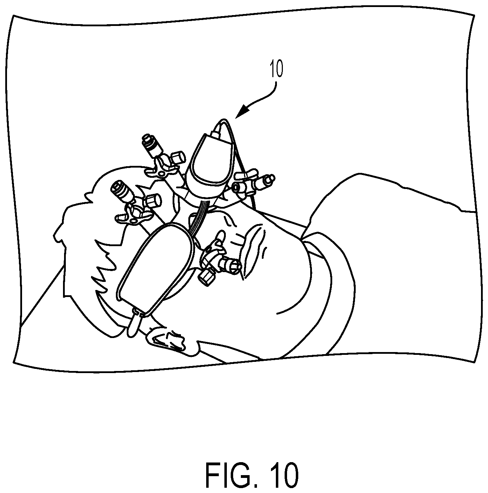

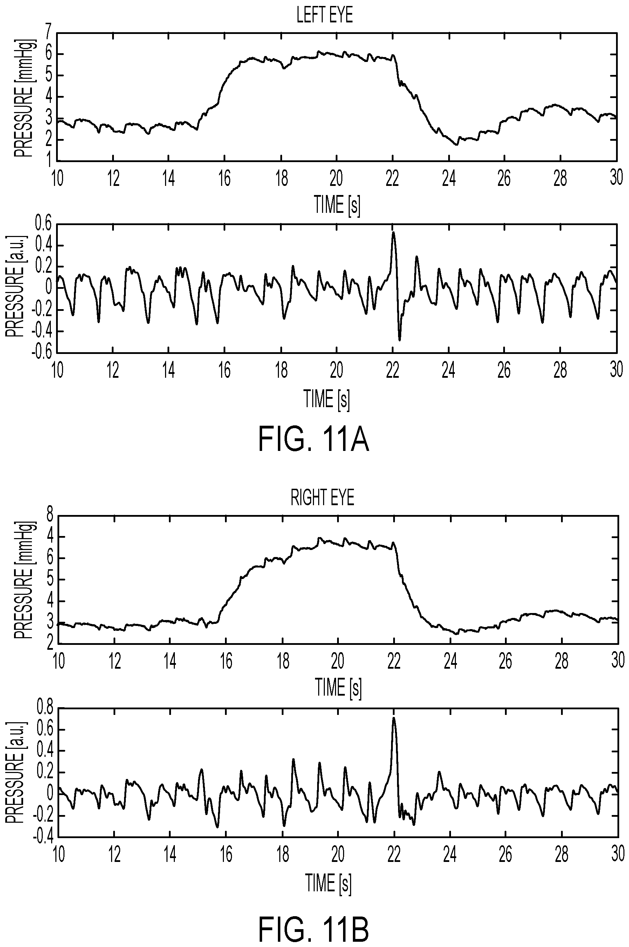

**Fig. 10****.** Shows a volunteer in a supine body position with the inventive monitor placed on the closed eyelids, during the Valsalva maneuver. **Figs. 11A** and **11B** show the intracranial pressure pulse waves acquired from the left eye, right brain hemisphere (A) and right eye, left brain hemisphere (B) of a healthy volunteer during a Valsalva maneuver using the inventive non-invasive pressure monitor illustrated in **Fig. 9****.**

In cases of severe traumatic brain injuries, it is not enough to maintain intracranial pressure below the patient-specific critical ICP threshold and to maintain optimal brain perfusion by monitoring brain autoregulation state indices. There is an increasing number of publications and prospective clinical studies, which show that it is necessary to introduce real-time intracranial compliance monitoring into multimodal monitoring systems. Non-invasive real-time monitoring of intracranial compliance is currently not possible in clinical practice. The invention soves the problem of non-invasive intracranial compliance monitoring using real-time algorithms for morphological analysis and complexity analysis of recorded pulse waves.

The invention also allows for the measurement of the magnitude of the pulsating CSF pressure in the subarachnoid space of the optic nerve. Using these measurements permits a better way to measure intracranial compliance. While it was know how to measure radial pulsation of the sheath of the optic nerve the invention allows for measurement of the axial pulsation. The invention contemplates using the inventive device in an inventive way by measuring the pulsation and converting the pulse of the CSF pressure that pulses axially through the optic nerve to measure intracranial compliance.

Real time monitoring of the pulse wave of CSF pressure can be used for many clinical purposes including; to cover the changes of intracranial compliance in brain trauma; to investigate glaucoma; to measure slow waves in cerebral autoregulation monitoring; to diagnose regrowing brain tumors, to monitor CSF pressure non-invasively during surgeries with general anesthesia (organ transplantation, cardiac surgery, etc.) and to investigate spaceflight associated neuro ocular syndrome.

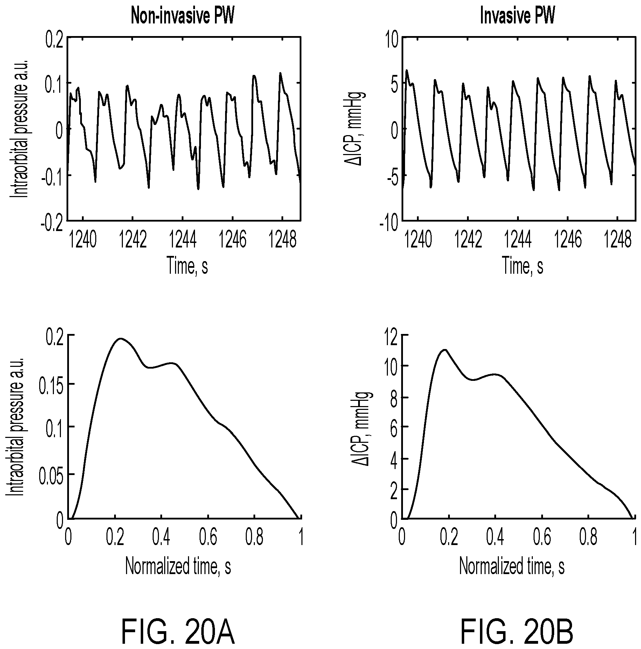

**Fig. 20** shows the comparison of non-invasive (a) and invasive ICP pulse waves (b). The raw monitored signals of simultaneously recorded non-invasive and invasive ICP pulse waves are shown in curves (upper graphs). The processed non-invasive and invasive ICP pulse waves, after eliminating slow and respiratory waves, normalization, and averaging, are shown in curves (bottom graphs). The invasive ICP pulse waves were recorded using the Raumedic Neurovent-PTO invasive ICP sensor implanted in the brain parenchyma. The correlation coefficient between averaged invasive and non-invasive pulse waves is r=0.993.

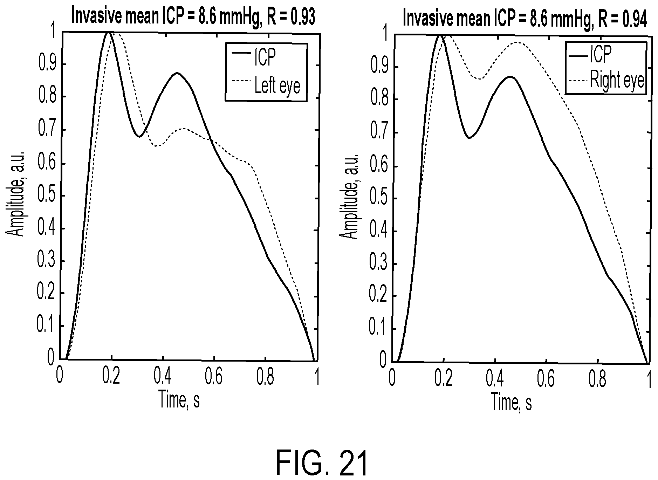

**Fig. 21** shows the measurements for a patient after traumatic brain injury (TBI). Invasive Raumedic ICP sensor was implanted to brain parenchyma ("ICP") and the inventive non-invasive ICP pulsatile monitor was monitored simultaneously in the left eye ("Left eye") and the right eye ("Right eye"). The averaged waves were normalized by maximum of amplitude. The mean ICP during recording was 8.6 mmHg and correlation between invasive ICP and the left eye was R = 0.93 and between the right eye was R = 0.94.

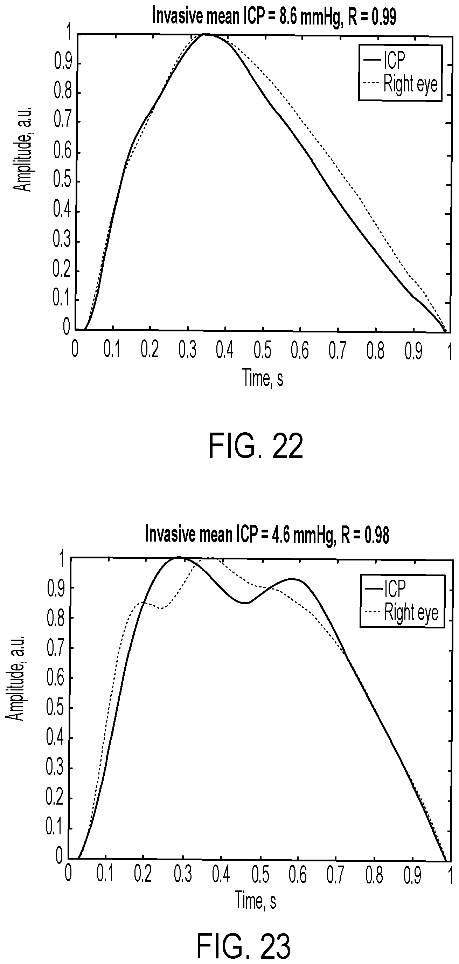

**Fig. 22** shows the measurements for a patient after subarachnoid hemorrhage (SAH). Invasive Raumedic ICP sensor was implanted to brain parenchyma ("ICP") and non-invasive ICP pulsatile monitor was monitored simultaneously in the right eye ("Right eye"). The averaged waves was normalized by maximum of amplitude. The mean ICP during recording was 8.6 mmHg and correlation between invasive ICP and the left eye was R = 0.93 and between the right eye was R = 0.99.

**Fig. 23** shows the measurement for a patient after traumatic brain injury (TBI). Invasive Raumedic ICP sensor was implanted to brain parenchyma ("ICP") and non-invasive ICP pulsatile monitor was monitored simultaneously in the right eye ("Right eye"). The averaged waves were normalized by maximum of amplitude. The mean ICP during recording was 4.6 mmHg and correlation between invasive ICP and the right eye was R = 0.98.

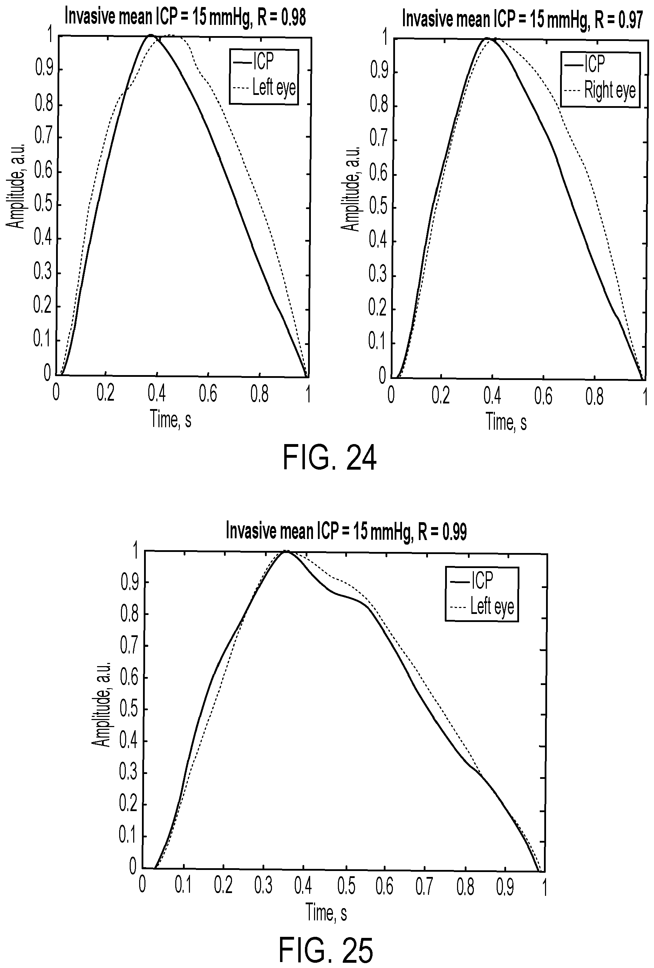

**Fig. 24** shows the measurements for a patient after traumatic brain injury (TBI). Invasive Raumedic ICP sensor was implanted to brain parenchyma ("ICP") and non-invasive ICP pulsatile monitor was monitored simultaneously in the left eye ("Left eye") and the right eye ("Right eye"). The averaged waves were normalized by maximum of amplitude. The mean ICP during recording was 15mmHg and correlation between invasive ICP and the left eye was R = 0.98 and between the right eye was R = 0.97.

**Fig. 25** shows the measurements for a patient after surgical brain tumor removal. Invasive Raumedic ICP sensor was implanted to brain parenchyma ("ICP") and non-invasive ICP pulsatile monitor was monitored simultaneously in left eye ("Left eye"). The averaged waves were normalized by maximum of amplitude. The mean ICP during recording was 15mmHg and correlation between invasive ICP and the left eye was R = 0.99.

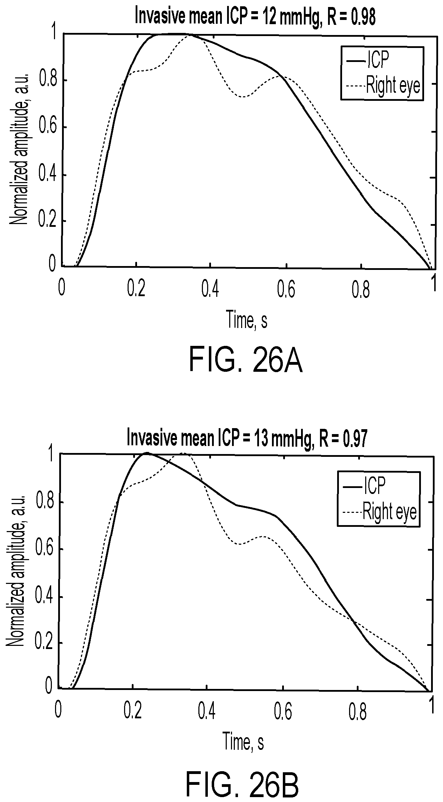

**Fig. 26** shows the measurements for a patient after traumatic brain injury (TBI) with different small compliance changes. Invasive Raumedic ICP sensor was implanted to brain parenchyma ("ICP") and non-invasive ICP pulsatile monitor was monitored simultaneously in the right eye ("Right eye"). The averaged waves were normalized by maximum of amplitude. a) The mean ICP during recording was 12 mmHg and correlation between the invasive ICP and the left eye was R = 0.98 with head up sitting position; b) The mean ICP during recording was 13 mmHg and correlation between invasive ICP and the left eye was R = 0.97 with little change in body position (from sitting position a little bit closer to supine) which demonstrates a small compliance decrement by decrement of amplitude of the ICP pulse wave second peak.

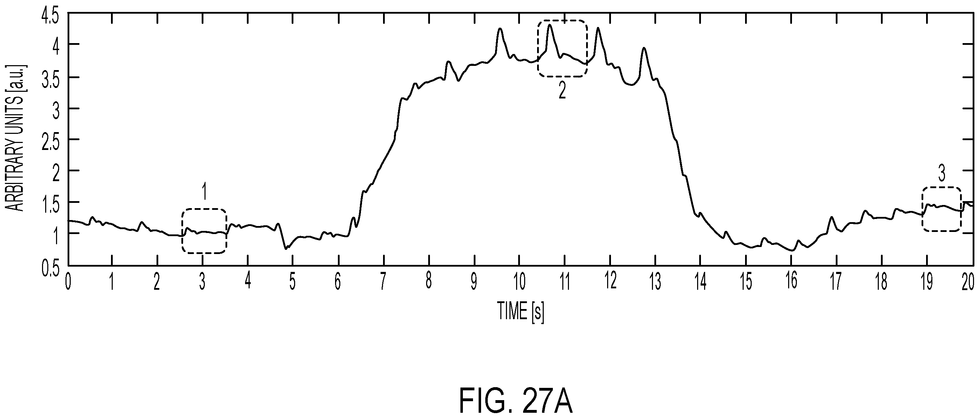

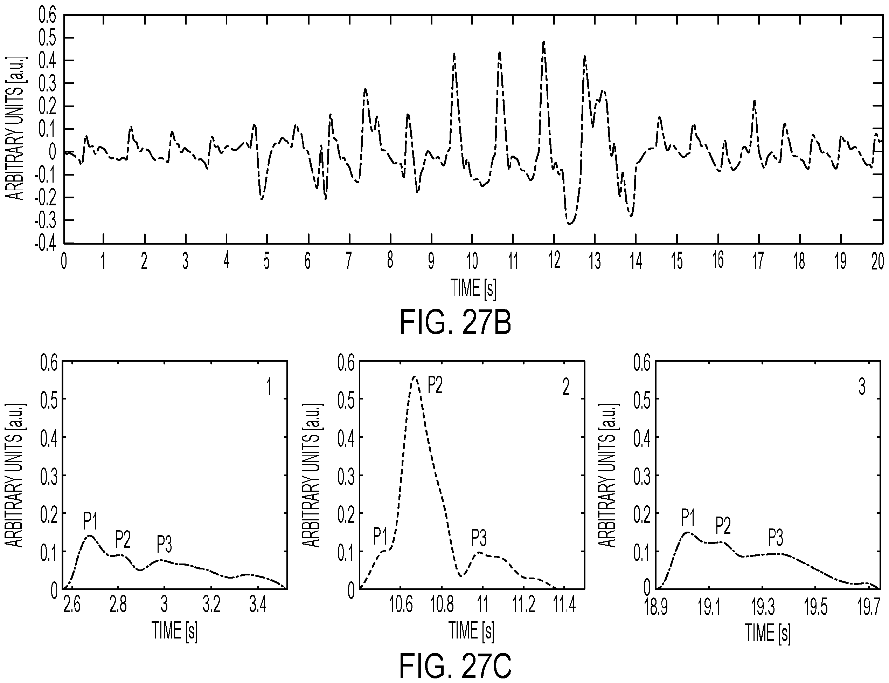

**Fig. 27** shows pressure pulse waves recorded using the inventive device during the Valsalva maneuver. **Fig. 27** **A** shows the raw signal data with three segmented pressure pulse waves marked by red dashed rectangular boxes: 1 shows before the Valsalva maneuver, 2 shows at the end of the maneuver, and 3 shows during recovery after the maneuver. **Fig. 27B** shows detrended data from the Valsalva maneuver. **Fig.27** **C** shows segmented pressure pulse waves with corresponding P2/P1 ratios: 1 shows a ratio of 0.64, 2 shows a ratio of 5.58, and 3 shows a ratio of 0.83. Pulse wave 2, recorded during the Valsalva maneuver, demonstrates decreased intracranial compliance and elevated intracranial pressure due to increased intracranial blood volume, resulting in a significantly elevated peak P2.

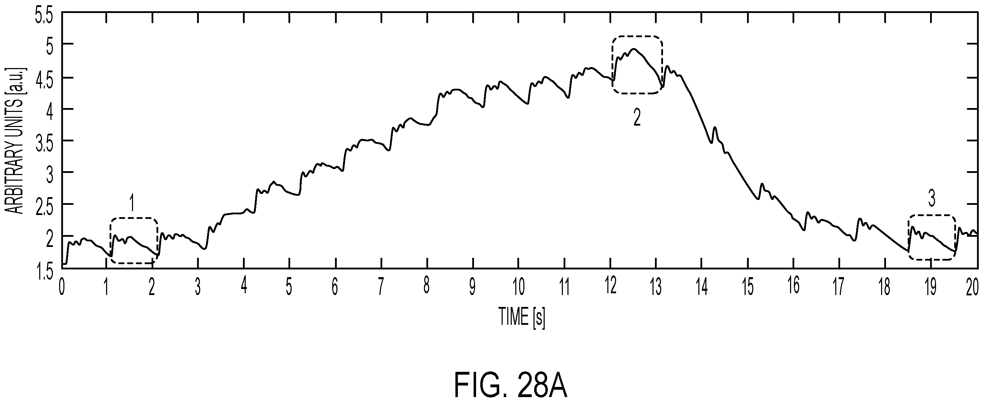

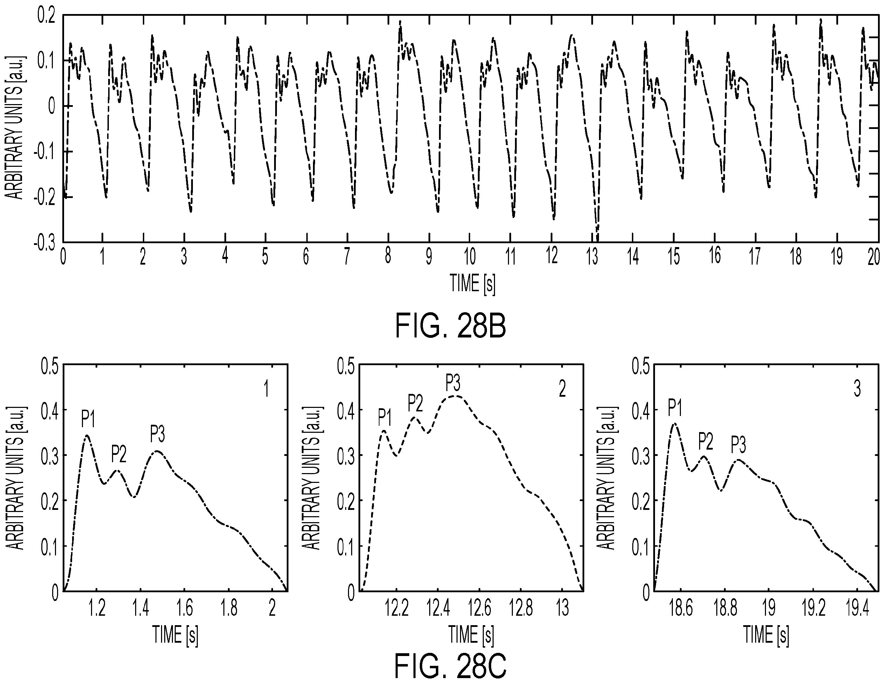

A typical pressure signal recorded during the Queckenstedt test is shown in **FIG 28****.** Ultrasound-guided compression of both jugular veins was performed on a healthy volunteer in the supine position for approximately 10 seconds using a PHILIPS EPIQ Elite ultrasound scanner.

**Fig 28** shows pressure pulse waves recorded using the inventive device during the Queckenstedt test. **Fig. 28A** shows raw signal data with three segmented pressure pulse waves marked by red dashed rectangular boxes: 1 show before the Queckenstedt maneuver, 2 shows at the end of the maneuver, and 3 shows during recovery after the maneuver. **Fig. 28** B shows detrended data from the Queckenstedt maneuver. **Fig. 28** C shows segmented pressure pulse waves with corresponding P2/P1 ratios: 1 - ratio of 0.78, 2 - ratio of 1.08, and 3 - ratio of 0.80. P3/P1 ratios are as follows: 1 - ratio of 0.89, 2 - ratio of 1.22, and 3 - ratio of 0.78. Pulse wave 2, recorded during the Queckenstedt maneuver, demonstrates decreased intracranial compliance due to increased intracranial blood volume, resulting in elevated P2 and P3 peaks and increased P3/P1 ratio because of cerebral venous outflow resistance increment during Queckenstedt test.

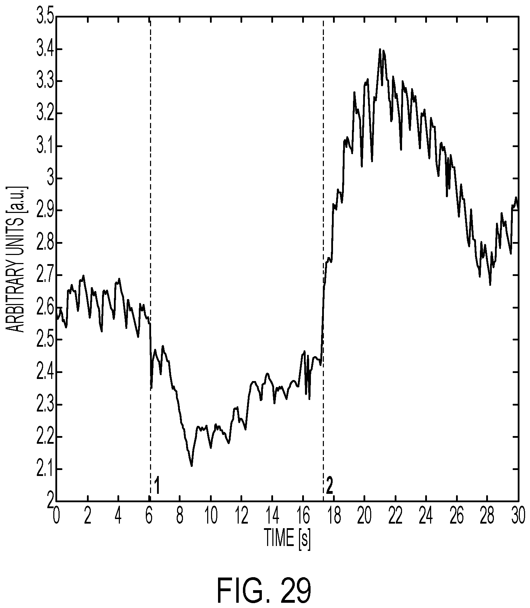

**Fig 29** shows the pressure pulse waves recorded using the inventive device during the Transient Hypoemic/Hyperemic Response test. 1 shows the fast compression of common carotid artery, 2 shows the fast release of compression. The cerebral autoregulation system's transient response to hypoemic test (after maneuver 1) demonstrates an intact cerebral blood flow autoregulation process, as indicated by the rising signal. The reaction to the hyperemia test (following maneuver 2) also demonstrates the typical transient response associated with intact cerebral blood flow autoregulation. Ultrasound-guided compression of the carotid artery was performed on a healthy volunteer in the upright position for approximately 10 seconds using a PHILIPS EPIQ Elite ultrasound scanner.

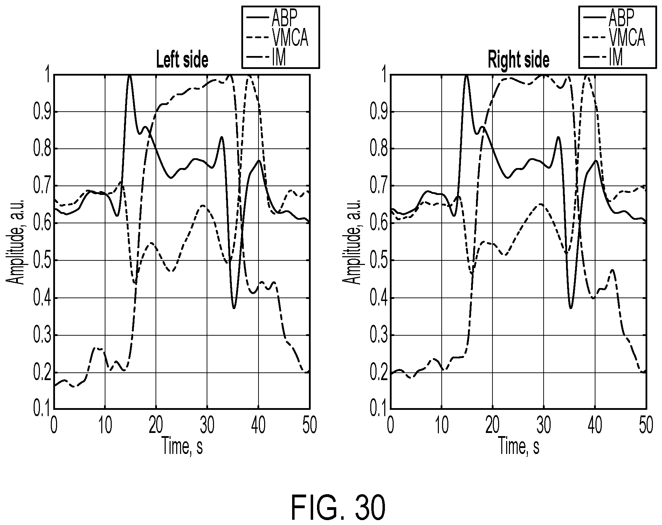

**Fig. 30** shows the simultaneous non-invasive multimodal signals monitoring during a Valsalva maneuver. Transient response of ICP signal recorded with the inventive device is depicted as "IM" transient response of arterial blood pressure (ABP) signal recorded with Finapres monitor is depicted as "ABP", and transient response of blood flow velocity in middle cerebral artery (VMCA) recorded with TCD monitor (Dolphin, Viasonix 4D) is depicted as "VCMA". All signals are visualized after normalization. At the beginning of Valsalva maneuver VMCA decreased since blood vessels expanded causing ICP to increase. At the end of a maneuver VMCA increased since blood vessels contracted causing ICP to decrease.

Intracranial pressure (ICP) pulse wave morphology, including the ratios of three peaks (P1, P2, P3), offers valuable insights into brain conditions. Traditional invasive methods for ICP pulse wave monitoring pose significant risks, highlighting the need for non-invasive alternatives.

The study involved ten volunteers, aged 26-39 years, who underwent non-invasive ICP wave monitoring using the inventive device and inventive method while the body of the volunteer was positioned supine. Recorded signals were processed to extract pulse waves and evaluate their characteristics. Six steps of signal processing are used to extract the pulse waves. The six steps of signal processing are used were: (1) Application of a third-order Butterworth bandpass filter with lower and upper -3 dB cutoff frequencies of 0.5 Hz and 8 Hz, respectively, to remove offsets, slow trends, and respiratory waves, and to extract pulse waves from the raw pressure signal: (2) Detection of diastolic points in the continuous pressure signal to extract each pulse wave separately: (3) Detrending each pulse wave individually to bring the values of the first and last points of each pulse wave to zero; (4) Interpolation and decimation of individual pulse waves to standardize each pulse wave to 100 data points; (5) Rejection of distorted pulse waves to include only valid ones in the final calculation of the average pulse wave; and (6) Detection of peaks P1, P2, and P3 from the averaged pulse wave.

Results indicated successful detection of pressure pulse waves with the expected three peaks in all subjects. The calculated P2/P1 ratios were 0.762 for the left eye and 0.808 for the right eye, suggesting normal intracranial compliance across the cohort, despite variations observed in some individuals. A Valsalva maneuver and a Queckenstedt test both demonstrated changes in the P2/P1 ratio, demonstrating the non-invasively recorded pressure pulse waves, measured through closed eyelids, reflect intracranial volume/pressure dynamics. A Transient Hypoemic/Hyperemic Response test carried out on a healthy brain induced changes in the signal recorded with the inventive device and method indicating intact cerebral blood flow autoregulation. The invention permits monitoring of ICP waves and ICP wave changes non-invasively through closed eyelids.

Intracranial pressure (ICP) pulse waveform and its parameters provide valuable diagnostic information about brain conditions. The amplitude of the ICP pulse wave depends primarily on the mean value and the pulse amplitude of the cerebral blood volume, depending on the pathology. The ICP pulse waveform is characterized not only by its amplitude but also by the presence of three distinct peaks: P1, P2, and P3. Other parameters include peak appearance time, rise time coefficient, downward coefficient, wave duration, area under curve, etc. Overall, 24 metrics can be extracted using an algorithm termed Morphological Clustering and Analysis of ICP Pulse (MOCAIP).

The first peak, P1, arises from the rapid expansion of the walls of the cerebral arteries in response to the systolic rise in arterial blood pressure. This expansion is transmitted to the cerebrospinal fluid (CSF) and other intracranial media and can thus be identified in the pressure signal. The second peak, P2, is associated with an increase in arterial intracranial blood volume. This volume increase induces pressure changes within the skull, thus the amplitude of P2 depends on the compliance of the intracranial compartment. Consequently, the ratio between P1 and P2 is considered indicative of intracranial compliance. The third peak, P3, is linked to cerebral venous blood outflow resistance.

In a healthy brain, P1 is higher than both P2 and P3, although not all these peaks are always visible in ICP pulse wave monitoring records. Changes in these peaks' ratios can indicate pathological conditions of the brain. For example, a decreased ratio between P1 and P2 indicates a decline in intracranial compliance.

Other non-invasive methods to monitor ICP waveforms exist, including those detecting small variations in skull deformation using a strain gauge sensor (B4C) placed over the skin of the temporal bone; detecting a brain photoplethysmographic (PPG) signal; measuring a head's dielectric properties with electrically isolated electrodes on the scalp and utilizing transcranial ultrasound to measure attenuation and time of flight of ultrasonic pulses to detect dynamical changes in the brain media.

What is needed is an improved way to monitor intracranial pressure waives non-invasively and entirely passively (without transmitting ultrasonic, electromagnetic, or other physical signals) through closed eyelids. The invention that meets this need is a liquid-filled, non-invasive, fully passive sensor and ICP pulse wave monitor that provides high temporal resolution and high sensitivity for pressure wave recording.

Due to its anatomical connection to the cerebrospinal fluid via the subarachnoid space of the optic nerve, the human eye can provide a non-invasive means of accessing information about CSF pressure dynamics Clinical studies suggest that spontaneous retinal venous pulsation is in phase with intracranial pressure and is likely related to the gradient between ICP and intraocular pressure waveforms. The optic nerve head also features pulsatile deformation. No prior art measures the spatial movement of the eyeball caused by ICP waves and changes in the subarachnoid space of the optic nerve.

The invention is based on the anatomical 'hydraulic pump' within the subarachnoid space of the optic nerve, caused by its cul-de-sac anatomy, which translates intracranial pressure to the optic nerve head and moves the eyeball in response to changes in ICP(t). The subtle ICP pulsations and changes, which move the eyeball, can be monitored through the closed eyelid using a highly sensitive pressure sensor and hydrostatic mechanical contact via a non-compressive liquid between the pulsating eyeball and the digital pressure sensor.

The anatomy of an eye ball connected to the optic nerve covered by dura mater is similar to the structure of a hydraulic pump. CSF movement inside the optic nerve sheath is similar to non-compressible fluid movement in an automobile brake system. To measure the axial pulsation of CSF in subarachnoid space around the optic nerve the gaze of the patients of should be kept close to straight in order to sense the physiological and interpretable close to axial pulsation of CSF in subarachnoid space around the optic nerve. For healthy patients the operator asks them to keep their gaze straight during monitoring. The straight gas is observed for unconscious patient after brain trauma or sedated patients.

Volunteers were initially placed in a supine position on a tilt table (Teeter Hang Ups Power II Inversion Table, Teeter, Bonney Lake, WA, USA). At first, arterial blood pressure (ABP) was measured with a Microlife (BP B6 Connect, Microlife, Widnau, Switzerland) ABP meter. An automatic triple measurement mode was used for each ABP measurement. Three measurements were automatically taken in succession, and the results were then automatically analysed and displayed. Then, the pressure monitor with both cups filled with liquid, was placed on the volunteer's closed eyelids. With this setup, the pressure signal was recorded on a laptop. For subjects who consented, a Valsalva maneuver was instructed. An ultrasound scanner/Doppler-guided Queckenstedt test and Transient Hypoemic/Hyperemic Response tests were also performed. Afterward, the pressure monitor was removed from the volunteer's head, and ABP was measured again at the end of the procedure.

An analysis was conducted to examine the occurrence of detected three peaks in averaged pressure pulse waves. The mean values ± SD (standard deviation) of the amplitude of averaged pulse waves and the ratio of P2/P1 were calculated from the data. Additionally, correlation coefficient was calculated between averaged pulse waves obtained from the right and left eyes of each volunteer to assess the similarity of signals recorded from both eyes for the same subject.

We have included 10 volunteers, 5 males and 5 females, in this pilot study. The average age (±SD) was 30.2 (±3.4) years (range: 26-39 years). The characteristics of the individual subjects are presented in Table 1. The mean (±SD) values at the beginning of the experiments were 124.9 (±8.1) mmHg for systolic blood pressure, 71.8 (±3.3) mmHg for diastolic blood pressure, and 69.6 (±15.9) beats/min for heart rate. Although the mean (±SD) systolic blood pressure and heart rate showed some reduction after the experimental procedures-to 121.7 (±10.4) mmHg and 64.9 beats/min, respectively-these changes were not significant. Mean diastolic blood pressure showed no change, remaining at 71.9 (±4.6) mmHg.

**Table 1. Characteristics of the volunteers included in this study.**

| **No.** | **Age, years** | **Gender** | **ABP before, mmHg** | | | **ABP after, mmHg** | | |
|---|---|---|---|---|---|---|---|---|
| | | | **SYS** | **DIAS** | **PULSE** | **SYS** | **DIAS** | **PULSE** |
| 1 | 29 | Male | 122 | 70 | 64 | 132 | 77 | 64 |
| 2 | 27 | Male | 141 | 69 | 52 | 144 | 72 | 54 |
| 3 | 26 | Female | 117 | 73 | 51 | 110 | 71 | 53 |
| 4 | 32 | Female | 128 | 75 | 66 | 120 | 74 | 68 |
| 5 | 31 | Male | 131 | 70 | 58 | 128 | 71 | 58 |
| 6 | 29 | Male | 132 | 76 | 107 | 121 | 75 | 86 |
| 7 | 39 | Male | 125 | 72 | 70 | 122 | 72 | 65 |
| 8 | 30 | Female | 120 | 77 | 86 | 119 | 78 | 78 |
| 9 | 30 | Female | 111 | 70 | 67 | 106 | 68 | 59 |
| 10 | 29 | Female | 122 | 66 | 75 | 115 | 61 | 64 |

ABP before: arterial blood pressure measured before the experimental procedures, ABP after: arterial blood pressure measured after the experimental procedures, SYS: systolic blood pressure, DIAS: diastolic blood pressure, PULSE: heart rate measured in beats per minute.

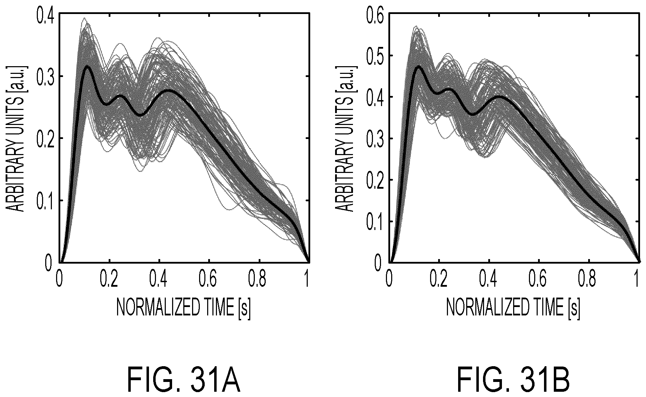

After the six step signal processing described above, pulse waves were successfully extracted from both eyes of all 10 subjects, resulting in 20 averaged pulse waves. **FIG. 31** shows noninvasively recorded valid pulse waves before averaging, along with the averaged pulse waves as thick black curves obtained from the left (31A) and right (31 B) eyes of the subject. Distinctly, three peaks can be identified in the recorded pressure waves from both the left and right eyes, with the second peak being lower than the first in both cases.

The thin curves represent the recorded pulse waves modulated by physiological ICP respiratory and slow waves. Since each individual pulse wave was standardized to 100 data points, the time axis is labeled as 'normalized time', and a duration of 1 second is assigned. Since filtering and detrending of the signals have been performed, the processed signal values are expressed in arbitrary units (a.u.).

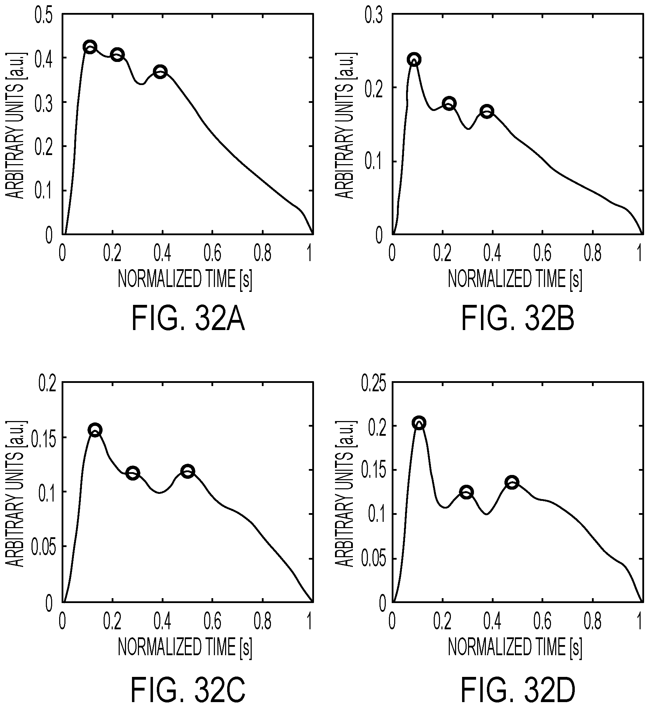

Three peaks have been detected in all 20 averaged pulse waves. Averaged pulse waves, along with the marked automatically detected three peaks, obtained from volunteers No. 2 and No. 10-who have normal but different intracraniospinal compliances-are shown in **Fig. 32****.**

**Fig 32****.** Shows examples of averaged pulse waves with three automatically detected peaks marked with circles. **Figs 32A, 32B** shows pulse waves recorded from subject No. 2's left and right eyes, respectively. **Figs 32C, 32D** shows pulse waves recorded from subject No. 10's left and right eyes, respectively. Since pulse waves were standardized to 100 data points, the time axis is labeled as 'normalized time', and a duration of 1 second is assigned. Since filtering and detrending of the signals have been performed, the processed signal values are expressed in arbitrary units (a.u.). Comparison of pulse waves shows that subject No. 2 has normal but lower compliance compared to subject No. 10.

Generalized results of the recorded pressure pulse waves for each subject individually are presented in Table 2. On average, pressure pulse waves were recorded for 3 minutes and 51 seconds. The mean (±SD) number of included pulse waves in the final calculations was 210 (±83) for the left eye and 194 (±66) for the right eye.

The mean (±SD) amplitude of the averaged pressure pulse waves was 0.217 (±0.103) a.u. for the left eye and 0.189 (±0.104) a.u. for the right eye, respectively. On average (±SD), the ratio between peaks P2 and P1 was 0.762 (±0.229) for the left eye and 0.808 (±0.310) for the right eye. Both mean values are below 1 and could indicate normal intracranial compliance.

The average (±SD) correlation coefficient R=0.804 (±0.267) showed a strong relationship between the pressure pulse waves recorded from the left and right eyes.

**Table 2. Results of the recorded pressure pulse waves.**

| **No.** | **Record duration** | **Number of valid pulse waves** | | **Amplitude (P1), a.u.** | | **Ratio of P2/P1** | | **R** |
|---|---|---|---|---|---|---|---|---|
| | | **Left** | **Right** | **Left** | **Right** | **Left** | **Right** | |
| 1 | 3 min. 1 sec. | 131 | 124 | 0.315 | 0.472 | 0.851 | 0.887 | 0.996 |
| 2 | 3 min. 12 sec. | 146 | 125 | 0.424 | 0.238 | 0.958 | 0.746 | 0.971 |
| 3 | 3 min. 0 sec. | 107 | 125 | 0.151 | 0.122 | 0.678 | 0.506 | 0.724 |
| 4 | 3 min. 1 sec. | 173 | 175 | 0.084 | 0.111 | 0.196 | 1.122 | 0.071 |
| 5 | 4 min. 3 sec. | 210 | 197 | 0.259 | 0.129 | 0.843 | 1.265 | 0.918 |
| 6 | 4 min. 20 sec. | 314 | 320 | 0.152 | 0.130 | 0.555 | 0.171 | 0.650 |
| 7 | 3 min. 4 sec. | 176 | 160 | 0.331 | 0.181 | 0.830 | 0.717 | 0.858 |
| 8 | 6 min. 24 sec. | 389 | 288 | 0.156 | 0.102 | 0.997 | 1.021 | 0.929 |
| 9 | 5 min. 1 sec. | 262 | 243 | 0.147 | 0.197 | 0.960 | 1.036 | 0.979 |
| 10 | 3 min. 17 sec. | 194 | 184 | 0.155 | 0.203 | 0.748 | 0.611 | 0.943 |

Record duration: the time span of the pressure pulse waves' recording, valid pulse waves: pulse waves included in the calculation of the average pulse wave, a.u.: arbitrary units, R: the correlation coefficient between averaged pulse waves obtained from the left and right eyes.

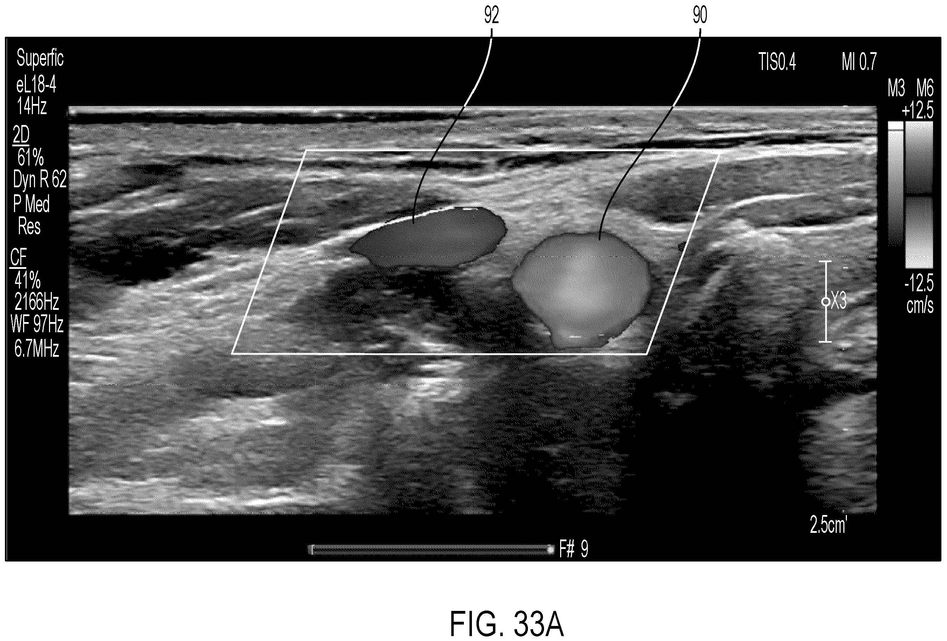

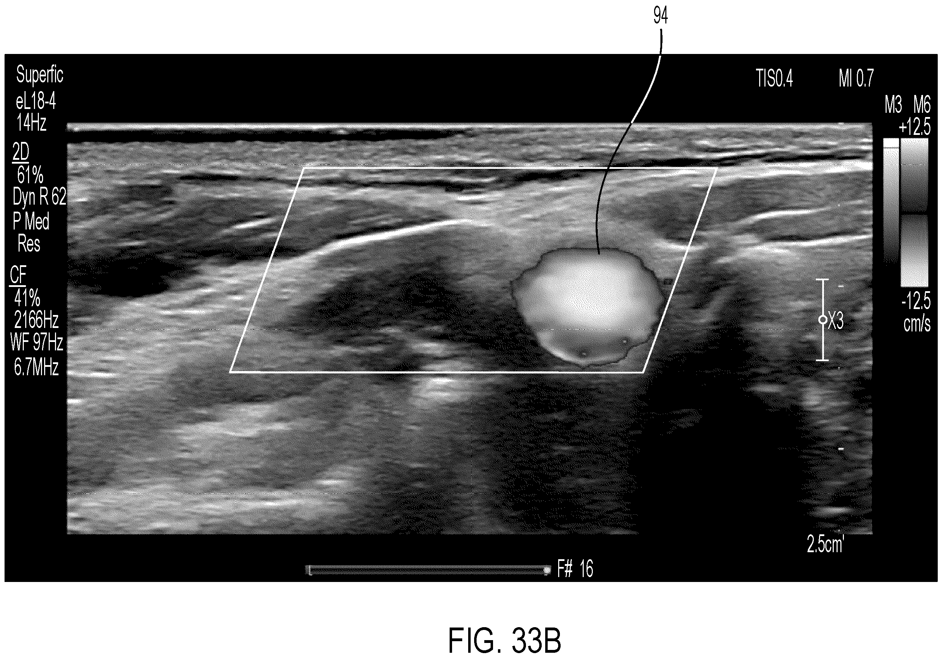

A typical pressure signal recorded non-invasively on a subject through a closed eyelid using the pressure monitor during the Valsalva maneuver is shown in **Fig. 33****.**

A typical pressure signal recorded during the Queckenstedt test is shown in **FIG 28****.** Ultrasound-guided compression of both jugular veins was performed on a healthy volunteer in the supine position for approximately 10 seconds using a PHILIPS EPIQ Elite ultrasound scanner **Fig. 33****.**

**Fig 33A** shows before a compression of the jugular veins, carotid artery blood flow **90** and jugular vein blood flow **92** are displayed on the screen of the PHILIPS EPIQ Elite ultrasound scanner. **Fig. 33B** shows no jugular vein blood flow visible on the screen due to compression of the jugular veins, only carotid artery blood flow **94.**

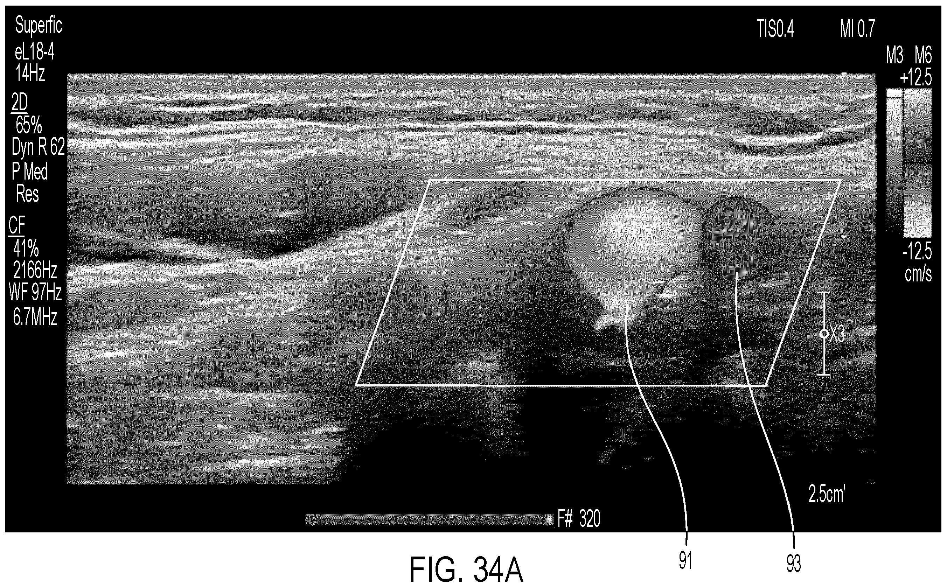

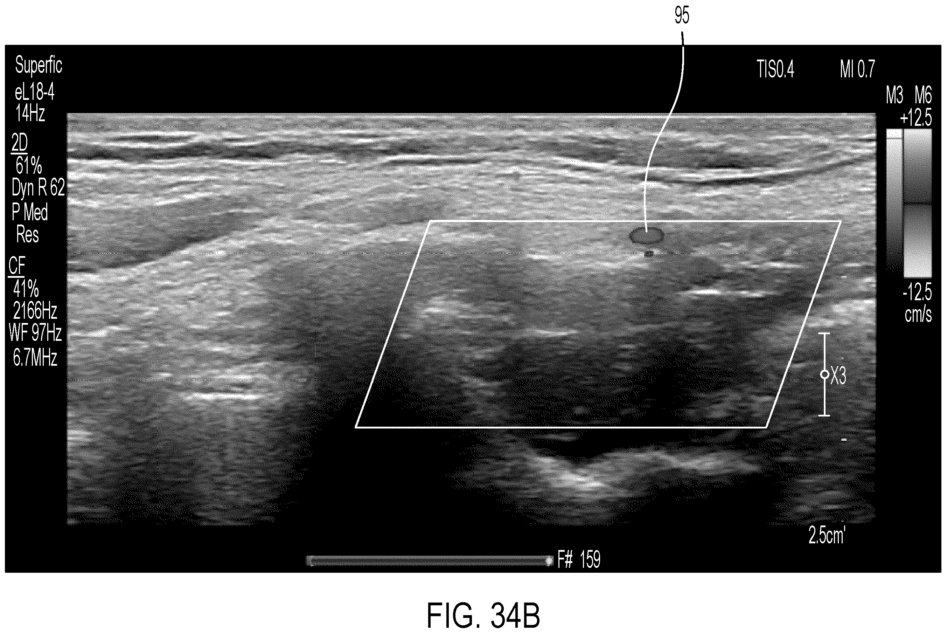

**Fig 34****.** Shows the screen of the PHILIPS EPIQ Elite ultrasound triplex scanner during the ultrasound-guided carotid artery compression test (Transient Hypoemic/Hyperemic Response test). **Fig 34A** shows before compression of the common carotid artery, the common carotid artery blood flow **91** and the jugular vein blood flow **93** are displayed on the PHILIPS EPIQ Elite ultrasound scanner. **Fig. 34B** shows during compression of the common carotid artery, minimal to no carotid artery blood flow 95 or jugular vein blood flow is visible on the screen. This test was performed in the upright position, when blood flow in the jugular veins is physiologically minimal.

The invention is an apparatus and method for sensing and measuring spatial eye movement associated with intracranial volume/pressure/compliance changes and waves. The Valsalva maneuver and Queckenstedt test experiments showed that the P2/P1 ratio of pulse waves recorded with the inventive device increased during these tests compared to the P2/P1 ratio before and after, indicating decreased intracranial compliance and elevated intracranial pressure due to increased intracranial blood volume. The results of these tests, along with the response observed during the Transient Hypoemic/Hyperemic Response test, show that the recorded reactions and pressure pulse waves from closed eyelids originate from intracranial pressure dynamics.

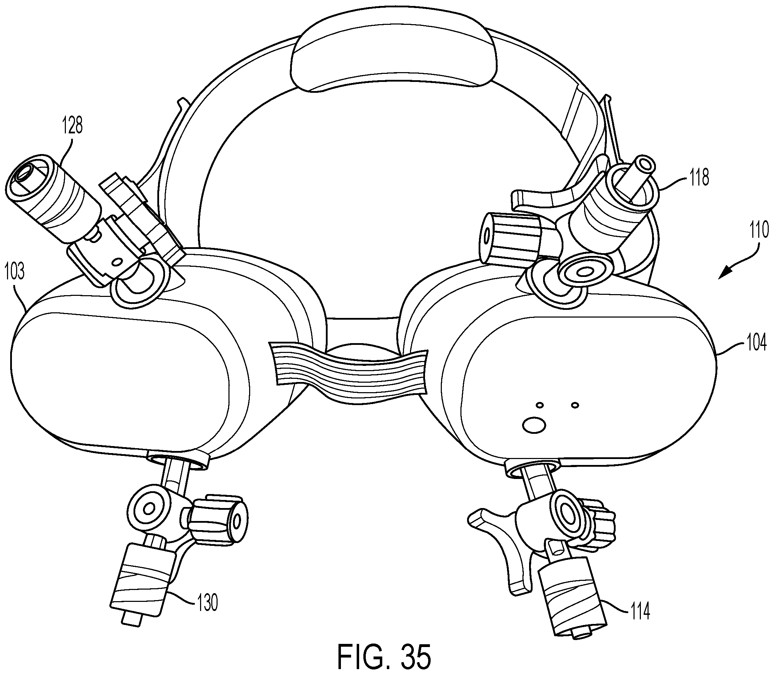

**FIG. 35** is a front perspective view of one embodient of the inventive intracranial pressure monitor 110 with the right sensor assembly **103,** with an upper valve **128,** lower valve **130** and the left sensor assembly **104** with a lower valve **114** and and upper valve **118.**

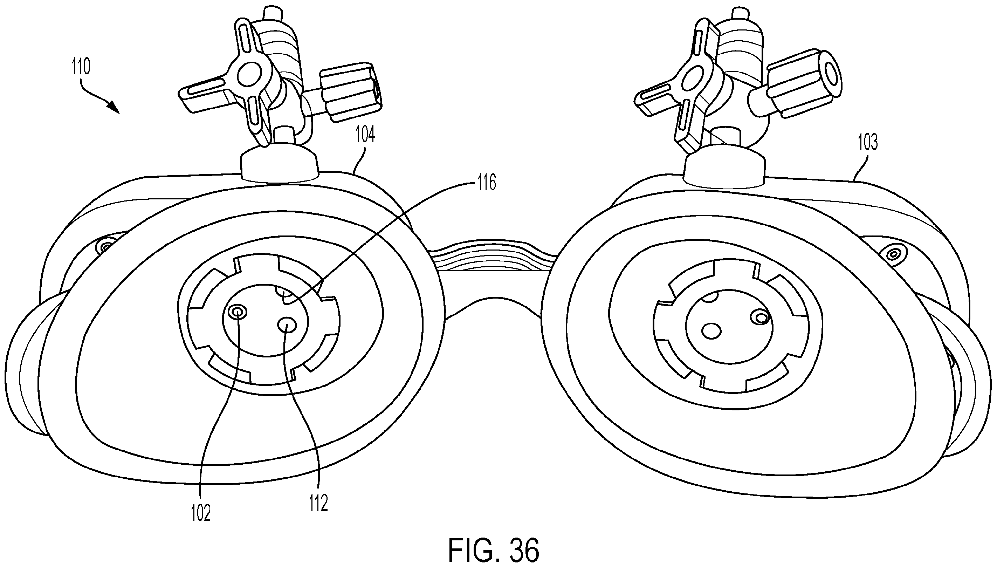

**Fig 36****.** is a rear view the pressure monitor **110** of **FIG 35****.** without a head band and without the silicon film. A sensor hole **102** in the side of the left sensor assembly **104** allows a stainless steel pressure sensor port, (not seen) to protrude from the sensor left sensor assembly **104** through the sensor hole **102** and into the hermetically sealed chamber of the pressure monitor **102,** enabling direct contact of the pressure sensor port with the liquid in the filled hermetically sealed chamber. A first valve hole **112** is also in the side of the left sensor assembly . The valve hole **112** connects to a lower valve **114,** not seen in the figure, used for filling the hermetically sealed chamber with liquid. Also in the side of the left sensor assembly is an upper valve hole **116.**

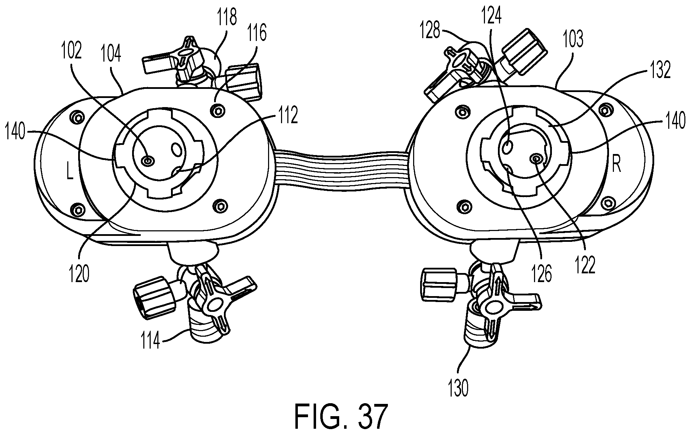

**Fig 37****.** is a rear view of the left sensor assembly **104,** and right sensor assembly **103.** The left sensor assembly **104** includes the sensor hole **102** that allows the stainless steel pressure sensor port to protrude from the sensor assembly 104 into the sealed chamber, enabling direct contact with the liquid when the chamber is filled. The valve hole **112** is connected to the lower valve **114** to the chamber and allow fluid to flow from the lower valve **114** into the sealed chamber with liquid. A third upper hole **116** connects an upper valve **118** to the chamber which is used for air removal. Either the lower valve **114** or upper valve **118** can be used for injecting liquid or removing air. The left sensor assembly **104** also has a locking ring **120** for attaching the to the right front sealing cover **134.**

The right sensor assembly **103** has a similar structure as the left sensor assembly **104.** It includes a sensor hole **122** to allow the pressure sensor port to protrude from the right sensor assembly **103** into a second sealed chamber and an upper valve hole **124** and a lower valve hole **126** for connecting to an upper valve **128** and a lower valve hole **126** for connecting to the lower valve **130.** The right sensor assembly **103** also has a locking ring **132** for attaching to the left front sealing cover **136.**

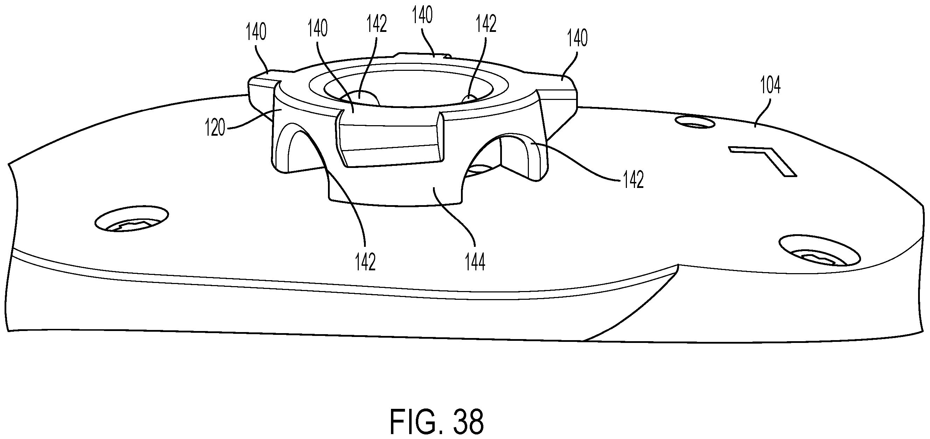

**FIG. 38** shows a side view perspective view of the locking ring **120** of the left sensor assembly **104** with a circular wall **144** and four tabs **140** that each mate with openings in the right front sealing cover 134 to form one of the water tight hermetically sealed chambers in the pulse wave monitor formed by the joining of the left sensor assembly **104** and the right front sealing cover **134.** The tabs **140** are located around the periphery of the top of the circular wall **144** approximately 90 degrees apart to form a strong clamping force and seal when fully assembled. Because of the physical features and characteristics of various locking mechanisms problems can arise due to the formation of air pockets or air bubbles within the sealed chamber when fluid is introduced into the chamber. Formation of air pockets or air bubbles within the sealed chamber can degrade the accuracy and precision of the pressure measurements by the pressure sensors within the fluid. The locking rings can include circular walls with apertures that allow fluid to flow through the circular walls. The apertures in the circular wall **144** help reduce air bubbles or air pockets from forming in the sealed chambers. For example, **FIG. 38** shows four archways **142** spaced apart 90 degrees around the circular wall **144** where it extends from the left sensor assembly **104** to form in the wall of the locking mechanism. In previous designs air bubbles would congregate around the locking mechanism. With the new design with arches for apertures the air bubbles are more easily removed from the chamber when filling the sealed chambers with fluid.

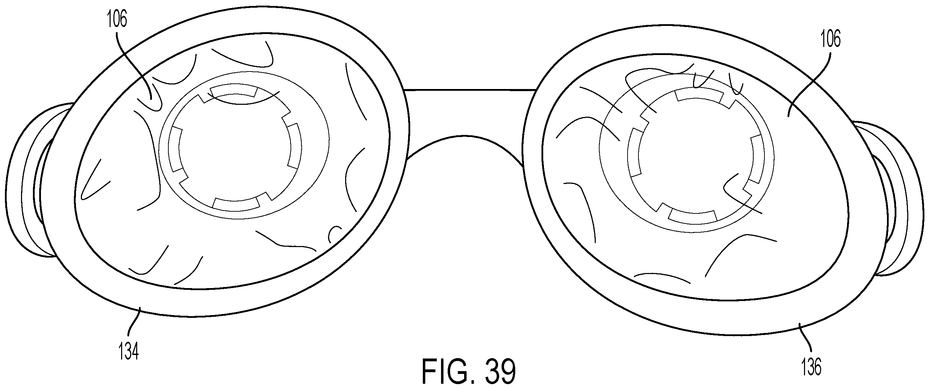

**FIG. 39** shows a rear view of the goggle assembly without the head strap of the right front sealing assembly **134** and left front sealing assembly **136** and the silicon film **106** that forms part of the hermetically sealed chambers in the google assembly.

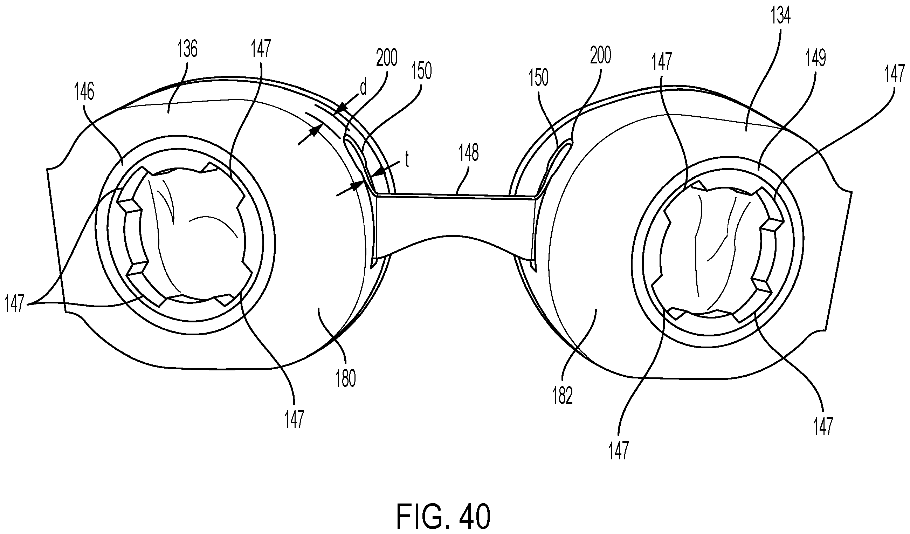

**FIG. 40** shows a front view of the goggle assembly without the head strap of the right front sealing cover **134** and left front sealing cover **136** before they are attached to the sensor assemblies **103** and **104.** The right front sealing cover **134** and left front sealing cover **136** each have an O-ring **146, 149** positioned in a groove on its face**180, 182.** The two sealing covers are connected by a flexible connector **148**. The edges of the flexible connector **148** slide into edge grooves **150** in the edges of the right front sealing cover **134** and left front sealing cover **136**. Tab openings **147** in the front sealing covers can also be seen in the figure. These tab openings **147** allow the tabs **140** of the locking ring **132** to engage and penetrate the faces **180, 182** of the right front sealing cover **134** and left front sealing cover **136** and engage internal ramps **152** inside the right front sealing cover **134** and left front sealing cover **136**.

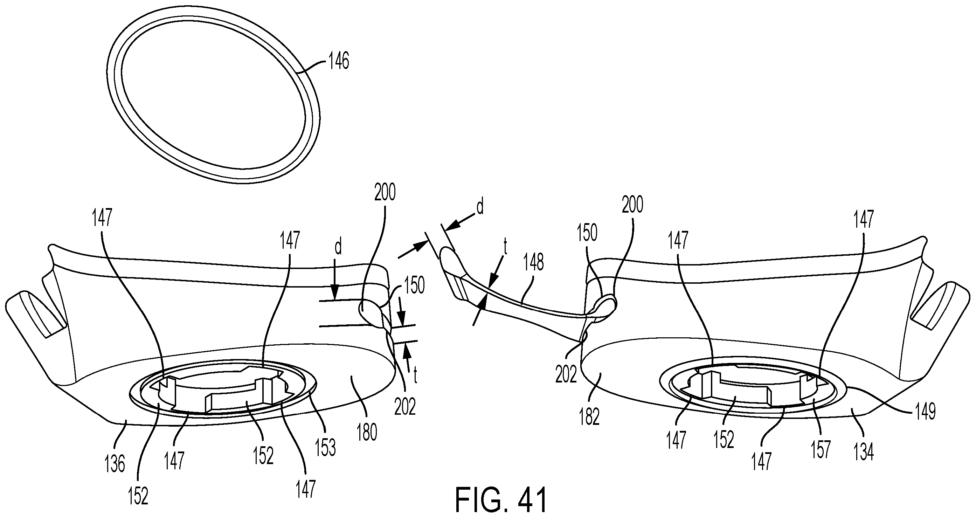

**FIG 41****.** is a top viewing showing right front sealing cover **134** and left front sealing cover **136** with the left front sealing cover **136** disconnected from the flexible connector **148** and with its the O- ring **146** removed from its mating groove **153** in the face **180** of the left front sealing cover **136**. From this perspective multiple tab openings **147** and ramps **152** can be seen inside the right front sealing cover **134** and left front sealing cover **136.** The tab openings **147** and ramps **152** are configured to permit a locking engagement with the tabs **140** and pull the front sealing covers **134** and **136,** towards the sensor assemblies **103, 104,** compressing the O-rings, **146, 149** and creating a hermetically sealed contact between the sensor assemblies **103, 104** and the front sealing covers **134, 136.** The locking engagement occurs with a rotational motion between the sensor assemblies **103, 104** and the front sealing assemblies **134, 136** after the tabs of the locking collars have been inserted to the tab openings **147** and engage the ramps **152.** The twisting motion cases the tabs of the locking collars to engage the ramps the of the front sealing assemblies to be pulled together and form the sealed chamber. Once in locking engagement the sensor assemblies and goggle assembly can be disengaged by rotating the sensor assemblies relative to the front sealing collars in the opposite direction from the engagement rotation. This allows for removeable and repeated engagement and disengagement between the sensor assemblies and different goggle assemblies.

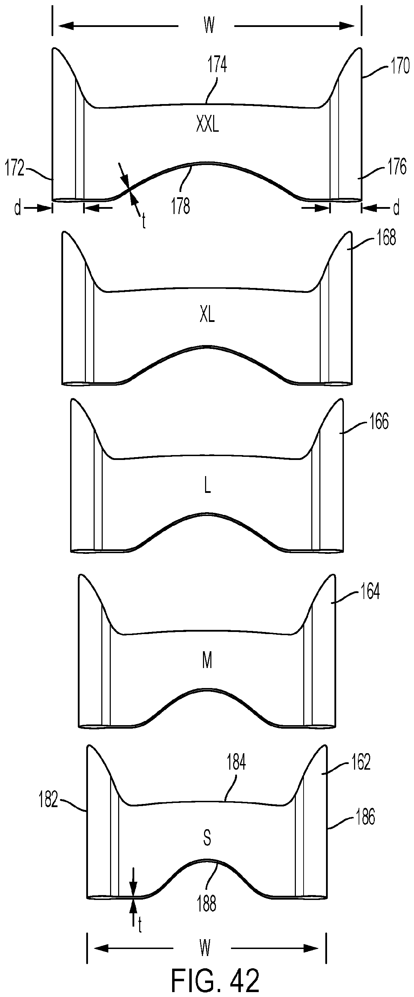

**FIG. 42** shows the front view of a set of various sized flexible connectors, a small connector **162,** a medium connector **164,** a large connector **166,** an extra-large connector **168** and an extra-extra-large connector **170.** All the flexible connectors share a common structure. For example, the extra-extra-large connector **170** has a first column **172,** a bridge portion **174** and a second column **176.** The lower edger of the flexible connector **170** has a curved radius **178** to adapt and fit over the bridge of the nose of the patient being monitored. The first and second columns **172, 176** are on opposite edges of the flexible connector **170** and configured and sized to slide easily in and out of grooves **150** on the right front sealing cover **134** and left front sealing cover **136.** Similarly, the small sized flexible connector **162** has a first column **182,** a bridge portion **184** and a second column **186.** The lower edger of the flexible connector **162** has a curved radius **188** to adapt and fit over the bridge of the nose of the patient being monitored. The first columns **172, 182** and second columns **176, 186** of the flexible connector have diameters d that are just slightly smaller than the diameter d of the top **200** of groove **150** and bridges **174, 184** that have thickness t slightly smaller than the thickness t of the side **202** of the grooves **150** that allow the flexible connector to be slid into the groove from an opening in the top **200** of the groove **150.**

Various sized flexible connectors are needed because the goggle system is designed to be used a single time of a variety of different sized people and must fit the person being monitored. As there is a difference in the facial structure of people to be monitored with the goggles and variations in the distance between peoples' eyes and the size of their nose, having multiple sized flexible connectors will allow for the google system to be custom fit to each patient to monitored. Preferably, the width "W" of the connector ranges between 27 mm to 35 mm in 2 mm increments. This preferred ranges of connectors will accommodate the vast majority of people to be monitored. A set of sized connectors can include a small connector **163,** a medium connector **164,** a large connector **164,** an extra-large connector **168** and an extra-extra-large connector **170** with the smallest connector having a width of 27 mm and the extra-extra-large connector having a width of 35 mm. The set of connectors share columns of substantial the same size so can be used interchangeably to connect the right side and left side sensor assemblies. The flexible connectors can be marked or formed with letters (e.g. S, M, L, XL, XXL) to help a technician or device operator to quickly identify and select the most appropriate size to use for a particular test subject and can be inserted into the grooves to connect the right front sealing cover **134** with the left front sealing cover **136.** One embodiment of the invention includes a goggle kit that includes a disposable goggle unit with five different sized flexible connectors. The kit allows the goggle unit to be customized and fitted for any patient to be monitored regardless of the size of the patient's facial features. The interchangeable connectors allow for quick and easy insertion and removal and allows the google unit to be quickly modified to custom fit the patient to monitored.

All of the components of the intracranial pressure monitor are made and configured to allow sterilization of all the components. The goggle system is designed to be disposable and the right side and left side sensor assemblies are configured for easy attachment and detachment from the goggle system to allow repeated use of the right side and left side sensor assemblies with many different goggle systems.

It should be noted that the invention in its broader aspects is not limited to the specific details, representative compositions, methods, and processes, and illustrative examples described in connection with the preferred embodiments and preferred methods. Modifications and equivalents will be apparent to practitioners skilled in this art and are encompassed within the spirit and scope of the appended claims. Figures are representative and not necessarily to scale.

## Claims

1. A non-invasive intracranial pulse wave monitor comprising:
a goggle system, a right side sensor assembly and a left side sensor assembly, the goggle system comprising a right front sealing cover, a left front sealing cover, a back right sealing cover, and a back left sealing cover;
a flexible connector for connecting the right front and left front sealing covers; an adjustable strap connected to the right front and left front sealing covers configured to adjust around a patient's head;
two silicon membranes, the first silicon member positioned between of the right front sealing cover and the back right sealing cover and the second silicon member positioned between the left front sealing cover and the back left sealing cover forming a water tight seals around the periphery edge of the silicon membranes;
the right side sensor assembly being having a first locking ring configured to form a first water tight chamber with the left front sealing cover and the left side sensor assembly having a second locking ring configured to form a second water tight chamber with the right front sealing cover when the sensor assemblies are engaged with the sealing covers;
the right side sensor assembly and left side sensor assembly each containing a pressure sensor that extends through the respective back cover of the sensor assembly into the water tight chamber and can measure pressure changes in the water tight chamber;
the right side sensor assembly having a first valve configured to allow water to be injected through the right side sensor assembly and into the first water tight chamber;
the left side sensor assembly having a second valve configured to allow water to be injected through the left side sensor assembly and into the second water tight chamber;
at least one of the right side sensor assembly and left side sensor assembly have a powered PCB board configured to transmit measurement data from at least one of the two pressure sensors wirelessly to a receiver adapted to process the measurement data for pulsating wave forms.

2. The pulse wave monitor of claim 1 wherein the left front sealing cover has four openings configured to mate with four tabs on the first locking ring and the right front sealing cover has four openings configured to mate with four tabs on the second locking ring.

3. The pulse wave monitor of claim 2 wherein the first locking ring comprises a circular wall extending outwardly from the right side sensor assembly and the second locking ring comprise a circular wall extending outwardly from the left side sensor assembly.

4. The pulse wave monitor of claim 3 wherein the locking rings comprise circular walls having apertures that allow fluid to flow through the circular walls.

5. The pulse wave monitor of claim 4 wherein the apertures are arches.

6. The pulse wave monitor of claim 1 wherein the monitor includes an interchangeable set of at least three different sized flexible connectors.

7. The non-invasive intracranial pulse wave monitor of claim 1 wherein the two water tight chambers are filled with water at a pressure between 1.0mmHg and 3.00mmHg.

8. The non-invasive intracranial pulse wave monitor of claim 1 wherein the measurement data transmission sent to a mobile device.

9. The non-invasive intracranial pulse wave monitor of claim 1 wherein the silicon membranes are between 40 microns to 50 microns.

10. The non-invasive intracranial pulse wave monitor of claim 1 wherein the pressure sensors are digital.

11. A noninvasive method for measuring intracranial pulse waves using the monitor of claim 1 comprising the steps of:
assembling the flexible connector between the right side sensor assembly and a left side sensor assembly;
locking the right side sensor assembly to the left front sealing cover to form the first water tight chamber and locking the left side sensor assembly having the second locking ring to the right front sealing cover to form the second water tight chamber;
positioning an intracranial pulse wave monitor on a patient's closed eyelids, the intracranial pulse wave monitor comprising a goggle system, a right side sensor assembly and a left side sensor assembly;
filling the water tight chambers with water;
measuring the pressure wave signals of the water within the water chamber with the pressure sensor;
transmitting the pressure signals to a processor for filtering out the intracranial pressure waves.

12. The method of claim 2 where in the pulse waves being monitored are the axial pulsation of CSF in the sheath of the optic nerve.
